# EUROPEAN PATENT SPECIFICATION

(11) **EP 3 432 864 B1**
(45) Date of publication and mention of the grant of the patent: **06.05.2020**
(21) Application number: 17713452.5
(22) Date of filing: 13.03.2017
(51) Int. Cl.: A61K 9/14, A61K 9/51

(54) **PROCESS FOR PREPARING THERAPEUTIC NANOPARTICLES**
VERFAHREN ZUR HERSTELLUNG THERAPEUTISCHER NANOPARTIKEL
PROCÉDÉ DE PRÉPARATION DE NANOPARTICULES THÉRAPEUTIQUES

(30) Priority: 22.03.2016 US 201662311624 P
(43) Date of publication of application: 30.01.2019
(73) Proprietor: Pfizer Inc., New York, NY 10017 (US)
(72) Inventor: HORHOTA, Allen, Westford, Massachusetts 01886 (US)
(74) Representative: Pfizer
(86) International application number: PCT/IB2017/051442
(87) International publication number: WO 2017/163145

(56) References cited:
- WO-A1-2015/161273
- US-A1- 2015 140 104

## Description

### BACKGROUND

Systems that deliver certain drugs to a patient (*e*.*g*., targeted to a particular tissue or cell type or targeted to a specific diseased tissue but not normal tissue) or that control release of drugs have long been recognized as beneficial.

For example, therapeutics that include an active drug and that are, *e.g.,* targeted to a particular tissue or cell type or targeted to a specific diseased tissue but not to normal tissue, may reduce the amount of the drug in tissues of the body that are not targeted. This is particularly important when treating a condition such as cancer where it is desirable that a cytotoxic dose of the drug is delivered to cancer cells without killing the surrounding non-cancerous tissue. Effective drug targeting may reduce the undesirable and sometimes life threatening side effects common in anticancer therapy. In addition, such therapeutics may allow drugs to reach certain tissues they would otherwise be unable to reach.

Therapeutics that offer controlled release therapy also must be able to deliver an effective amount of drug, which is a known limitation in other nanoparticle delivery systems. For example, it can be a challenge to prepare nanoparticle systems that have an appropriate amount of drug associated with each nanoparticle, while keeping the size of the nanoparticles small enough to have advantageous delivery properties.

Accordingly, a need exists for nanoparticle therapeutics and methods of making such nanoparticles that are capable of delivering therapeutic levels of the therapeutic agent to treat diseases such as cancer, while also reducing patient side effects.

### SUMMARY

The present disclosure generally relates to a process for preparing therapeutic nanoparticles, where the process includes combining a therapeutic agent with a substantially hydrophobic acid having at least some water solubility. The therapeutic nanoparticles may have, for example, improved drug loading and/or drug release properties.

In one aspect, a method of preparing a plurality of therapeutic nanoparticles is provided. The method comprises combining a first polymer and a therapeutic agent with an organic solvent to form a first organic phase having about 1 to about 50% solids; combining the first organic phase with a first aqueous phase to form an emulsion phase; combining the emulsion phase with a quench to form a quenched phase comprising the plurality of therapeutic nanoparticles, wherein the quench comprises water and a substantially hydrophobic acid having at least some water solubility; and recovering the therapeutic nanoparticles by filtration.

In some embodiments, the substantially hydrophobic acid is selected from the group consisting of caproic acid, enanthic acid, caprylic acid, pelargonic acid, capric acid, undecanoic acid, lauric acid, tridecanoic acid, myristic acid, pentadecanoic acid, palmitic acid, cinnamic acid, phenylacetic acid, dodecylbenzenesulfonic acid, dioctyl sulfosuccinic acid, dioleoyl phosphatidic acid, and chenodeoxycholic acid, ursodeoxycholic acid, deoxycholic acid, cholic acid, and lithocholic acid, and combinations thereof.

In some embodiments, contemplated therapeutic nanoparticles comprise about 0.05 to about 35 weight percent of the substantially hydrophobic acid.

In some embodiments, the molar ratio of the substantially hydrophobic acid to the therapeutic agent in the contemplated nanoparticle is about 0.1:1 to about 1.2:1, or about 0.1:1 to about 1:1, or about 0.1:1 to about 0.75:1, or about 0.75:1 to about 1.2:1.

In some embodiments, the first organic phase comprises a substantially hydrophobic acid having at least some water solubility. In some embodiments, the first aqueous phase comprises a substantially hydrophobic acid having at least some water solubility. In some embodiments, the emulsion phase comprises a substantially hydrophobic acid having at least some water solubility. In some embodiments, the molar ratio of the substantially hydrophobic acid to the therapeutic agent in the emulsion phase is about 0.5:1 to about 6:1, or about 1:1 to about 4:1.

In some embodiments, the method has an encapsulation efficiency of between about 75% and about 100%.

In some embodiments, contemplated therapeutic nanoparticles comprise about 5 to about 20 weight percent of the therapeutic agent, or about 10 to about 20 weight percent of the therapeutic agent, or about 10 to about 30 weight percent of the therapeutic agent.

In some embodiments, the quench has a temperature of about 0 °C to about 5°C.

In some embodiments, the quench:emulsion ratio is about 2:1 to about 40:1.

In some embodiments, contemplated methods for preparing therapeutic nanoparticles further comprise adding a drug solubilizer to the quenched phase to form a solubilized phase of unencapsulated therapeutic agent. In some embodiments, the drug solubilizer is selected from the group consisting of Tween 80, Tween 20, polyvinyl pyrrolidone, cyclodextran, sodium dodecyl sulfate, sodium cholate, diethylnitrosamine, sodium acetate, urea, glycerin, propylene glycol, glycofurol, poly(ethylene)glycol, bis(polyoxyethyleneglycol)dodecyl ether, sodium benzoate, and sodium salicylate.

In some embodiments, filtration comprises sterile filtration.

In some embodiments, contemplated therapeutic nanoparticles substantially immediately release less than about 5% of the therapeutic agent when placed in a phosphate buffer solution at 25°C. In some embodiments, contemplated therapeutic nanoparticles release about 0.01 to about 10% of the therapeutic agent over about 1 hour when placed in a phosphate buffer solution at 25°C. In some embodiments, contemplated therapeutic nanoparticles release about 10 to about 30% of the therapeutic agent over about 24 hours when placed in a phosphate buffer solution at 25°C.

In some embodiments, contemplated therapeutic nanoparticles have a diameter of about 60 nm to about 150 nm.

In some embodiments, the first polymer comprises a diblock poly(lactic) acid-poly(ethylene)glycol copolymer or a diblock poly(lactic acid-co-glycolic acid)-poly(ethylene)glycol copolymer.

In some embodiments, contemplated therapeutic nanoparticles comprise about 10 to about 30 weight percent poly(ethylene)glycol.

In some embodiments, the poly(lactic) acid-poly(ethylene)glycol copolymer has a poly(lactic) acid number average molecular weight fraction of about 0.7 to about 0.9. In some embodiments, the poly(lactic) acid-poly(ethylene)glycol copolymer has a poly(lactic) acid number average molecular weight fraction of about 0.75 to about 0.85.

In some embodiments, the poly(lactic) acid-poly(ethylene)glycol copolymer has a number average molecular weight of about 15kDa to about 20kDa poly(lactic acid) and a number average molecular weight of about 4kDa to about 6kDa poly(ethylene)glycol. In some embodiments, the poly(lactic) acid-poly(ethylene)glycol copolymer has a number average molecular weight of about 16kDa poly(lactic acid) and a number average molecular weight of about 5kDa poly(ethylene)glycol.

In another aspect, a plurality of therapeutic nanoparticles are provided. The therapeutic nanoparticles are prepared by a process comprising combining a first polymer and a therapeutic agent with an organic solvent to form a first organic phase having about 1 to about 50% solids; combining the first organic phase with a first aqueous phase to form an emulsion phase; combining the emulsion phase with a quench to form a quenched phase comprising the plurality of therapeutic nanoparticles, wherein the quench comprises water and a substantially hydrophobic acid having at least some water solubility; and recovering the therapeutic nanoparticles by filtration.

### BRIEF DESCRIPTION OF THE DRAWINGS

FIG. 1 is flow chart for an emulsion process for forming a disclosed nanoparticle.
FIGs. 2A and 2B show flow diagrams for a disclosed emulsion process.
FIG. 3A shows vincristine (VCR) drug loading and output ratio of octanoate to vincristine (*i.e*., ion pairing of octanoate with VCR) versus the ratio of octanoate to vincristine used in the organic phase, according to certain embodiments. FIG. 3B shows the amount of VCR released at 4 hours from contemplated nanoparticle formulations versus output ratio of octanoate to vincristine, according to certain embodiments. FIG. 3C shows the cumulative amount of VCR released over time from contemplated nanoparticle formulations, according to certain embodiments.
FIG. 4A shows vincristine (VCR) drug loading versus octanoic acid concentration in the quench, according to certain embodiments. FIG. 4B shows the cumulative amount of VCR released over time from contemplated nanoparticle formulations, according to certain embodiments.
FIG. 5A shows vincristine (VCR) drug loading versus hexanoic acid concentration in the quench, according to certain embodiments. FIG. 5B shows the cumulative amount of VCR released over time from contemplated nanoparticle formulations, according to certain embodiments.
FIG. 6 shows the cumulative amount of VCR released over time from contemplated nanoparticle formulations, according to certain embodiments.

### DETAILED DESCRIPTION

The present disclosure generally relates to a process for preparing therapeutic nanoparticles, where the process includes combining a therapeutic agent with a substantially hydrophobic acid having at least some water solubility. The therapeutic nanoparticles may have, for example, improved drug loading and/or drug release properties. In some embodiments, inclusion (*i.e*., doping) of a substantially hydrophobic acid in a disclosed nanoparticle and/or included in a nanoparticle preparation process may result in nanoparticles that include improved drug loading. Furthermore, in certain embodiments, nanoparticles that include and/or are prepared in the presence of the hydrophobic acid may exhibit improved controlled release properties. For example, disclosed nanoparticles may more slowly release the therapeutic agent as compared to nanoparticles prepared in the absence of the hydrophobic acid.

Without wishing to be bound by any theory, it is believed that the disclosed nanoparticle formulations that include a hydrophobic acid have significantly improved formulation properties (*e*.*g*., drug loading and/or release profile) through formation of a hydrophobic ion-pair (HIP), between the substantially hydrophobic acid and, *e*.*g*., the amine group of the therapeutic agent. As used herein, a HIP is a pair of oppositely charged ions held together by Coulombic attraction. Also without wishing to be bound by any theory, in some embodiments, a HIP can be used to increase the hydrophobicity of the therapeutic agent. In some embodiments, a therapeutic agent with increased hydrophobicity can be beneficial for nanoparticle formulations and result in HIP formation that may provide higher solubility of the therapeutic agent in organic solvents. HIP formation, as contemplated herein, can result in nanoparticles having for example, increased drug loading. Slower release of the therapeutic agent from the nanoparticles may also occur, for example in some embodiments, due to a decrease in the therapeutic agent's solubility in aqueous solution. Furthermore, complexing the therapeutic agent with large hydrophobic counter ions may slow diffusion of the therapeutic agent within the polymeric matrix. Advantageously, HIP formation occurs without the need for covalent conjugation of the hydrophobic group to the therapeutic agent.

Without wishing to be bound by any theory, it is believed that the strength of the HIP impacts the drug load and release rate of the contemplated nanoparticles. For example, the strength of the HIP may be increased by increasing the magnitude of the difference between the pKₐ of the therapeutic agent and the pKₐ of the hydrophobic acid, as discussed in more detail below. Also without wishing to be bound by any theory, it is believed that the conditions for ion pair formation impact the drug load and release rate of the contemplated nanoparticles.

Nanoparticles disclosed herein include one, two, three or more biocompatible and/or biodegradable polymers. For example, a contemplated nanoparticle may include about 35 to about 99.75 weight percent, in some embodiments about 50 to about 99.75 weight percent, in some embodiments about 50 to about 99.5 weight percent, in some embodiments about 50 to about 99 weight percent, in some embodiments about 50 to about 98 weight percent, in some embodiments about 50 to about 97 weight percent, in some embodiments about 50 to about 97.95 weight percent, in some embodiments about 50 to about 96 weight percent, in some embodiments about 50 to about 95 weight percent, in some embodiments about 50 to about 94 weight percent, in some embodiments about 50 to about 93 weight percent, in some embodiments about 50 to about 92 weight percent, in some embodiments about 50 to about 91 weight percent, in some embodiments about 50 to about 90 weight percent, in some embodiments about 50 to about 85 weight percent, in some embodiments about 50 to about 80 weight percent, and in some embodiments about 65 to about 85 weight percent of one or more block copolymers that include a biodegradable polymer and poly(ethylene glycol) (PEG), and about 0 to about 50 weight percent of a biodegradable homopolymer.

In some embodiments, the disclosed nanoparticles include a therapeutic agent, where the therapeutic agent is selected from vincristine and pharmaceutically acceptable salts thereof (vincristine pKₐ values: pKₐ₁ = 5.0; pKₐ₂ = 7.4).

In some embodiments, disclosed nanoparticles may include about 0.2 to about 35 weight percent, about 0.2 to about 20 weight percent, about 0.2 to about 10 weight percent, about 0.2 to about 5 weight percent, about 0.5 to about 5 weight percent, about 0.75 to about 5 weight percent, about 1 to about 5 weight percent, about 2 to about 5 weight percent, about 3 to about 5 weight percent, about 1 to about 20 weight percent, about 2 to about 20 weight percent, about 5 to about 20 weight percent, about 1 to about 15 weight percent, about 2 to about 15 weight percent, about 3 to about 15 weight percent, about 4 to about 15 weight percent, about 5 to about 15 weight percent, about 1 to about 10 weight percent, about 2 to about 10 weight percent, about 3 to about 10 weight percent, about 4 to about 10 weight percent, about 5 to about 10 weight percent, about 10 to about 30 weight percent, or about 15 to about 25 weight percent of the therapeutic agent.

In certain embodiments, disclosed nanoparticles comprise a hydrophobic acid and/or are prepared by a process that includes a hydrophobic acid. Such nanoparticles may have a higher drug loading than nanoparticles prepared by a process without a hydrophobic acid. For example, drug loading (*e.g*., by weight) of disclosed nanoparticles prepared by a process comprising the hydrophobic acid may be between about 2 times to about 10 times higher, or even more, than disclosed nanoparticles prepared by a process without the hydrophobic acid. In some embodiments, the drug loading (by weight) of disclosed nanoparticles prepared by a first process comprising the hydrophobic acid may be at least about 2 times higher, at least about 3 times higher, at least about 4 times higher, at least about 5 times higher, or at least about 10 times higher than disclosed nanoparticles prepared by a second process, where the second process is identical to the first process except that the second process does not include the hydrophobic acid.

Any suitable hydrophobic acid is contemplated. In some embodiments, the hydrophobic acid may be a carboxylic acid (*e.g*., a monocarboxylic acid, dicarboxylic acid, tricarboxylic acid, or the like), a sulfinic acid, a sulfenic acid, or a sulfonic acid. In some cases, a contemplated hydrophobic acid may include a mixture of two or more acids. In some cases, a salt of a hydrophobic acid may be used in a formulation.

For example, a disclosed carboxylic acid may be an aliphatic carboxylic acid (*e.g*., a carboxylic acid having a cyclic or acyclic, branched or unbranched, hydrocarbon chain). Disclosed carboxylic acids may, in some embodiments, be substituted with one or more functional groups including, but not limited to, halogen (*i.e*., F, Cl, Br, and I), sulfonyl, nitro, and oxo. In certain embodiments, a disclosed carboxylic acid may be unsubstituted.

Exemplary carboxylic acids may include a substituted or unsubstituted fatty acid (*e.g*., C₆-C₅₀ fatty acid). In some instances, the fatty acid may be a C₁₀-C₂₀ fatty acid. In other instances, the fatty acid may be a C₁₅-C₂₀ fatty acid. The fatty acid may, in some cases, be saturated. In other embodiments, the fatty acid may be unsaturated. For instance, the fatty acid may be a monounsaturated fatty acid or a polyunsaturated fatty acid. In some embodiments, a double bond of an unsaturated fatty acid group can be in the *cis* conformation. In some embodiments, a double bond of an unsaturated fatty acid can be in the *trans* conformation. Unsaturated fatty acids include, but are not limited to, omega-3, omega-6, and omega-9 fatty acids.

Non-limiting examples of saturated fatty acids include caproic acid, enanthic acid, caprylic acid, pelargonic acid, capric acid, undecanoic acid, lauric acid, tridecanoic acid, myristic acid, pentadecanoic acid, palmitic acid, margaric acid, stearic acid, nonadecanoic acid, arachidic acid, heneicosanoic acid, behenic acid, tricosanoic acid, lignoceric acid, pentacosanoic acid, cerotic acid, heptacosanoic acid, montanic acid, nonacosanoic acid, melissic acid, henatriacontanoic acid, lacceroic acid, psyllic acid, geddic acid, ceroplastic acid, hexatriacontanoic acid, and combinations thereof.

Non-limiting examples of unsaturated fatty acids include hexadecatrienoic acid, alpha-linolenic acid, stearidonic acid, eicosatrienoic acid, eicosatetraenoic acid, eicosapentaenoic acid, heneicosapentaenoic acid, docosapentaenoic acid, docosahexaenoic acid, tetracosapentaenoic acid, tetracosahexaenoic acid, linoleic acid, gamma-linolenic acid, eicosadienoic acid, dihomo-gamma-linolenic acid, arachidonic acid, docosadienoic acid, adrenic acid, docosapentaenoic acid, tetracosatetraenoic acid, tetracosapentaenoic acid, oleic acid (pKₐ = ∼4-5; logP = 6.78), eicosenoic acid, mead acid, erucic acid, nervonic acid, rumenic acid, α-calendic acid, β-calendic acid, jacaric acid, α-eleostearic acid, β-eleostearic acid, catalpic acid, punicic acid, rumelenic acid, α-parinaric acid, β-parinaric acid, bosseopentaenoic acid, pinolenic acid, podocarpic acid, palmitoleic acid, vaccenic acid, gadoleic acid, erucic acid, and combinations thereof.

Other non-limiting examples of hydrophobic acids include aromatic acids, such as 1-hydroxy-2-naphthoic acid (*i.e*., xinafoic acid) (pKₐ = ∼2-3; log P = 2.97), naphthalene-1,5-disulfonic acid (pKₐ = -2; logP = 1.3), naphthalene-2-sulfonic acid (pKₐ = -1.8; logP = 2.1), pamoic acid (pKₐ = 2.4), cinnamic acid, phenylacetic acid, (±)-camphor-10-sulfonic acid, dodecylbenzenesulfonic acid (pKₐ = -1.8; logP = 6.6), and combinations thereof. Other non-limiting examples of hydrophobic acids include dodecylsulfuric acid (pKₐ = -0.09; logP = 4.5), dioctyl sulfosuccinic acid (pKₐ = -0.8; logP = 5.2), dioleoyl phosphatidic acid (pKₐ = ∼2), and Vitamin D₃-sulfate (pKₐ = -1.5).

In some embodiments, the hydrophobic acid may be a bile acid. Non-limiting examples of bile acids include chenodeoxycholic acid, ursodeoxycholic acid, deoxycholic acid (pKₐ = 4.65; logP = 3.79), hycholic acid, beta-muricholic acid, cholic acid (pKₐ = ∼4.5; logP = 2.48), taurocholic acid, cholesteryl sulfate (pKₐ = -1.4), lithocholic acid, an amino acid-conjugated bile acid, and combinations thereof. An amino-acid conjugated bile acid may be conjugated to any suitable amino acid. In some embodiments, the amino acid-conjugated bile acid is a glycine-conjugated bile acid or a taurine-conjugated bile acid.

In certain instances, the hydrophobic acid may be a polyelectrolyte. For example, the polyelectrolyte may be a polysulfonic acid (e.g., poly(styrene sulfonic acid) or dextran sulfate) or a polycarboxylic acid (e.g., polyacrylic acid or polymethacrylic acid).

In some instances, a contemplated acid may have a molecular weight of less than about 1000 Da, in some embodiments less than about 500 Da, in some embodiments less than about 400 Da, in some embodiments less than about 300 Da, in some embodiments less than about 250 Da, in some embodiments less than about 200 Da, and in some embodiments less than about 150 Da. In some cases, the acid may have a molecular weight of between about 100 Da and about 1000 Da, in some embodiments between about 200 Da and about 800 Da, in some embodiments between about 200 Da and about 600 Da, in some embodiments between about 100 Da and about 300 Da, in some embodiments between about 200 Da and about 400 Da, in some embodiments between about 300 Da and about 500 Da, and in some embodiments between about 300 Da and about 1000 Da. In certain embodiments, a contemplated acid may have a molecular weight of greater than about 300 Da, in some embodiments greater than 400 Da, and in some embodiments greater than 500 Da. In certain embodiments, the release rate of a therapeutic agent from a nanoparticle can be slowed by increasing the molecular weight of the hydrophobic acid used in the nanoparticle formulation.

In some embodiments, a hydrophobic acid may be chosen, at least in part, on the basis of the strength of the acid. For example, the hydrophobic acid may have an acid dissociation constant in water (pKₐ) of about -5 to about 7, in some embodiments about -3 to about 5, in some embodiments about -3 to about 4, in some embodiments about -3 to about 3.5, in some embodiments about -3 to about 3, in some embodiments about -3 to about 2, in some embodiments about -3 to about 1, in some embodiments about -3 to about 0.5, in some embodiments about -0.5 to about 0.5, in some embodiments about 1 to about 7, in some embodiments about 2 to about 7, in some embodiments about 3 to about 7, in some embodiments about 4 to about 6, in some embodiments about 4 to about 5.5, in some embodiments about 4 to about 5, and in some embodiments about 4.5 to about 5, determined at 25°C. In some embodiments, the acid may have a pKₐ of less than about 7, less than about 5, less than about 3.5, less than about 3, less than about 2, less than about 1, or less than about 0, determined at 25°C.

In certain embodiments, the hydrophobic acid may be chosen, at least in part, on the basis of the difference between the pKₐ of the hydrophobic acid and the pKₐ of a protonated nitrogen-containing therapeutic agent. For example, in some instances, the difference between the pKₐ of the hydrophobic acid and the pKₐ of a protonated nitrogen-containing therapeutic agent may be between about 1 pKₐ unit and about 15 pKₐ units, in some embodiments between about 1 pKₐ unit and about 10 pKₐ units, in some embodiments between about 1 pKₐ unit and about 5 pKₐ units, in some embodiments between about 1 pKₐ unit and about 3 pKₐ units, in some embodiments between about 1 pKₐ unit and about 2 pKₐ units, in some embodiments between about 2 pKₐ units and about 15 pKₐ units, in some embodiments between about 2 pKₐ units and about 10 pKₐ units, in some embodiments between about 2 pKₐ units and about 5 pKₐ units, in some embodiments between about 2 pKₐ units and about 3 pKₐ units, in some embodiments between about 3 pKₐ units and about 15 pKₐ units, in some embodiments between about 3 pKₐ units and about 10 pKₐ units, in some embodiments between about 3 pKₐ units and about 5 pKₐ units, in some embodiments between about 4 pKₐ units and about 15 pKₐ units, in some embodiments between about 4 pKₐ units and about 10 pKₐ units, in some embodiments between about 4 pKₐ units and about 6 pKₐ units, in some embodiments between about 5 pKₐ units and about 15 pKₐ units, in some embodiments between about 5 pKₐ units and about 10 pKₐ units, in some embodiments between about 5 pKₐ units and about 7 pKₐ units, in some embodiments between about 7 pKₐ units and about 15 pKₐ units, in some embodiments between about 7 pKₐ units and about 9 pKₐ units, in some embodiments between about 9 pKₐ units and about 15 pKₐ units, in some embodiments between about 9 pKₐ units and about 11 pKₐ units, in some embodiments between about 11 pKₐ units and about 13 pKₐ units, and in some embodiments between about 13 pKₐ units and about 15 pKₐ units, determined at 25°C.

In some instances, the difference between the pKₐ of the hydrophobic acid and the pKₐ of a protonated nitrogen-containing therapeutic agent may be at least about 1 pKₐ unit, in some embodiments at least about 2 pKₐ units, in some embodiments at least about 3 pKₐ units, in some embodiments at least about 4 pKₐ units, in some embodiments at least about 5 pKₐ units, in some embodiments at least about 6 pKₐ units, in some embodiments at least about 7 pKₐ units, in some embodiments at least about 8 pKₐ units, in some embodiments at least about 9 pKₐ units, in some embodiments at least about 10 pKₐ units, and in some embodiments at least about 15 pKₐ units, determined at 25°C.

In some embodiments, the hydrophobic acid may have a logP of between about 2 and about 15, in some embodiments between about 5 and about 15, in some embodiments between about 5 and about 10, in some embodiments between about 2 and about 8, in some embodiments between about 4 and about 8, in some embodiments between about 2 and about 7, or in some embodiments between about 4 and about 7. In some instances, the hydrophobic acid may have a logP greater than about 2, greater than about 4, greater than about 5, or greater than 6.

In some embodiments, a contemplated hydrophobic acid may have a phase transition temperature that is advantageous, for example, for improving the properties of the therapeutic nanoparticles. For instance, the acid may have a melting point of less than about 300 °C, in some cases less than about 100 °C, and in some cases less than about 50°C. In certain embodiments, the acid may have a melting point of between about 5°C and about 25°C, in some cases between about 15 °C and about 50°C, in some cases between about 30 °C and about 100 °C, in some cases between about 75°C and about 150 °C, in some cases between about 125 °C and about 200 °C, in some cases between about 150 °C and about 250 °C, and in some cases between about 200 °C and about 300 °C. In some cases, the acid may have a melting point of less than about 15°C, in some cases less than about 10 °C, or in some cases less than about 0 °C. In certain embodiments, the acid may have a melting point of between about -30°C and about 0 °C or in some cases between about -20°C and about -10 °C.

For example, an acid for use in methods and nanoparticles disclosed herein may be chosen, at least in part, on the basis of the solubility of the therapeutic agent in a solvent comprising the acid. For example, in some embodiments, the therapeutic agent dissolved in a solvent comprising the acid may have a solubility of between about 15 mg/mL to about 200 mg/mL, between about 20 mg/mL to about 200 mg/mL, between about 25 mg/mL to about 200 mg/mL, between about 50 mg/mL to about 200 mg/mL, between about 75 mg/mL to about 200 mg/mL, between about 100 mg/mL to about 200 mg/mL, between about 125 mg/mL to about 175 mg/mL, between about 15 mg/mL to about 50 mg/mL, between about 25 mg/mL to about 75 mg/mL. In some embodiments, the therapeutic agent dissolved in a solvent comprising the acid may have a solubility greater than about 10 mg/mL, greater than about 50 mg/mL, or greater than about 100 mg/mL. In some embodiments, the therapeutic agent dissolved in a solvent comprising the hydrophobic acid (*e.g*., a first solution consisting of the therapeutic agent, solvent, and hydrophobic acid) may have a solubility of at least about 2 times greater, in some embodiments at least about 5 times greater, in some embodiments at least about 10 times greater, in some embodiments at least about 20 times greater, in some embodiments about 2 times to about 20 times greater or in some embodiments about 10 times to about 20 times greater than when the therapeutic agent is dissolved in a solvent that does not contain the hydrophobic acid (*e.g*., a second solution consisting of the therapeutic agent and the solvent).

In some instances, the concentration of acid in a drug solution (*i.e*., solution of therapeutic agent) may be between about 1 weight percent and about 30 weight percent, in some embodiments between about 2 weight percent and about 30 weight percent, in some embodiments between about 3 weight percent and about 30 weight percent, in some embodiments between about 4 weight percent and about 30 weight percent, in some embodiments between about 5 weight percent and about 30 weight percent, in some embodiments between about 6 weight percent and about 30 weight percent, in some embodiments between about 8 weight percent and about 30 weight percent, in some embodiments between about 10 weight percent and about 30 weight percent, in some embodiments between about 12 weight percent and about 30 weight percent, in some embodiments between about 14 weight percent and about 30 weight percent, in some embodiments between about 16 weight percent and about 30 weight percent, in some embodiments between about 1 weight percent and about 5 weight percent, in some embodiments between about 3 weight percent and about 9 weight percent, in some embodiments between about 6 weight percent and about 12 weight percent, in some embodiments between about 9 weight percent and about 15 weight percent, in some embodiments between about 12 weight percent and about 18 weight percent, and in some embodiments between about 15 weight percent and about 21 weight percent. In certain embodiments, the concentration of hydrophobic acid in a drug solution may be at least about 1 weight percent, in some embodiments at least about 2 weight percent, in some embodiments at least about 3 weight percent, in some embodiments at least about 5 weight percent, in some embodiments at least about 10 weight percent, in some embodiments at least about 15 weight percent, and in some embodiments at least about 20 weight percent.

In certain embodiments, the molar ratio of hydrophobic acid to therapeutic agent (*e.g*., initially during formulation of the nanoparticles, *e.g.*, in the aqueous phase, organic phase, emulsion phase, and/or quench, and/or in the nanoparticles) may be between about 0.1:1 to about 6:1, in some embodiments between about 0.1:1 to about 5:1, in some embodiments between about 0.1:1 to about 4:1, in some embodiments between about 0.1:1 to about 3:1, in some embodiments between about 0.1:1 to about 2:1, in some embodiments between about 0.1:1 to about 1.5:1, in some embodiments between about 0.1:1 to about 1.2:1, in some embodiments between about 0.1:1 to about 1:1, in some embodiments between about 0.1:1 to about 0.75:1, in some embodiments between about 0.1:1 to about 0.5:1, between about 0.25:1 to about 6:1, in some embodiments between about 0.25:1 to about 5:1, in some embodiments between about 0.25:1 to about 4:1, in some embodiments between about 0.25:1 to about 3:1, in some embodiments between about 0.25:1 to about 2:1, in some embodiments between about 0.25:1 to about 1.5:1, in some embodiments between about 0.25:1 to about 1:1, in some embodiments between about 0.25:1 to about 0.5:1, in some embodiments between about 0.5:1 to about 6:1, in some embodiments between about 0.5:1 to about 5:1, in some embodiments between about 0.5:1 to about 4:1, in some embodiments between about 0.5:1 to about 3:1, in some embodiments between about 0.5:1 to about 2:1, in some embodiments between about 0.5:1 to about 1.5:1, in some embodiments between about 0.5:1 to about 1:1, in some embodiments between about 0.5:1 to about 0.75:1, in some embodiments between about 0.75:1 to about 2:1, in some embodiments between about 0.75:1 to about 1.5:1, in some embodiments between about 0.75:1 to about 1.25:1, in some embodiments between about 0.75:1 to about 1:1, in some embodiments between about 1:1 to about 6:1, in some embodiments between about 1:1 to about 5:1, in some embodiments between about 1:1 to about 4:1, in some embodiments between about 1:1 to about 3:1, in some embodiments between about 1:1 to about 2:1, in some embodiments between about 1:1 to about 1.5:1, in some embodiments between about 1.5:1 to about 6:1, in some embodiments between about 1.5:1 to about 5:1, in some embodiments between about 1.5:1 to about 4:1, in some embodiments between about 1.5:1 to about 3:1, in some embodiments between about 2:1 to about 6:1, in some embodiments between about 2:1 to about 4:1, in some embodiments between about 3:1 to about 6:1, in some embodiments between about 3:1 to about 5:1, and in some embodiments between about 4:1 to about 6:1.

In some instances, the initial molar ratio of hydrophobic acid to therapeutic agent (*i.e*., during formulation of the nanoparticles) may be different from the molar ratio of hydrophobic acid to therapeutic agent in the nanoparticles (*i.e*., after removal of unencapsulated hydrophobic acid and therapeutic agent). In other instances, the initial molar ratio of hydrophobic acid to therapeutic agent (*i.e*., during formulation of the nanoparticles) may be essentially the same as the molar ratio of hydrophobic acid to therapeutic agent in the nanoparticles (*i.e*., after removal of unencapsulated hydrophobic acid and therapeutic agent).

In some cases, a solution containing the therapeutic agent may be prepared separately from a solution containing the polymer, and the two solutions may then be combined prior to nanoparticle formulation. For instance, in one embodiment, a first solution contains the therapeutic agent and the hydrophobic acid, and a second solution contains the polymer and optionally the hydrophobic acid. Formulations where the second solution does not contain the hydrophobic acid may be advantageous, for example, for minimizing the amount of hydrophobic acid used in a process or, in some cases, for minimizing contact time between the hydrophobic acid and, *e.g.*, a polymer that can degrade in the presence of the hydrophobic acid. In other cases, a single solution may be prepared containing the therapeutic agent, polymer, and hydrophobic acid.

In some embodiments, the hydrophobic ion pair may be formed prior to formulation of the nanoparticles. For example, a solution containing the hydrophobic ion pair may be prepared prior to formulating the contemplated nanoparticles (*e*.*g*., by preparing a solution containing suitable amounts of the therapeutic agent and the hydrophobic acid). In other embodiments, the hydrophobic ion pair may be formed during formulation of the nanoparticles. For example, a first solution containing the therapeutic agent and a second solution containing the hydrophobic acid may be combined during a process step for preparing the nanoparticles (*e*.*g*., prior to emulsion formation and/or during emulation formation). In certain embodiments, the hydrophobic ion pair may form prior to encapsulation of the therapeutic agent and hydrophobic acid in a contemplated nanoparticle. In other embodiments, the hydrophobic ion pair may form in the nanoparticle, *e*.*g*., after encapsulation of the therapeutic agent and hydrophobic acid.

In certain embodiments, the hydrophobic acid may have a solubility of less than about 2 g per 100 mL of water, in some embodiments less than about 1 g per 100 mL of water, in some embodiments less than about 100 mg per 100 mL of water, in some embodiments less than about 50 mg per 100 mL of water, in some embodiments less than about 25 mg per 100 mL of water, in some embodiments less than about 10 mg per 100 mL of water, in some embodiments less than about 5 mg per 100 mL of water, in some embodiments less than about 2 mg per 100 mL of water, and in some embodiments less than about 1 mg per 100 mL of water, determined at 25°C. In other embodiments, the acid may have a solubility of between about 1 mg per 100 mL of water to about 2 g per 100 mL of water, in some embodiments between about 1 mg per 100 mL of water to about 1 g per 100 mL of water, in some embodiments between about 1 mg per 100 mL of water to about 500 mg per 100 mL of water, in some embodiments between about 1 mg per 100 mL of water to about 100 mg per 100 mL of water, in some embodiments between about 1 mg per 100 mL of water to about 50 mg per 100 mL of water, in some embodiments between about 1 mg per 100 mL of water to about 25 mg per 100 mL of water, in some embodiments between about 1 mg per 100 mL of water to about 10 mg per 100 mL of water, in some embodiments between about 1 mg per 100 mL of water to about 5 mg per 100 mL of water, determined at 25°C. In some embodiments, the hydrophobic acid may be essentially insoluble in water at 25 °C.

In some embodiments, disclosed nanoparticles, once prepared, may be essentially free of any hydrophobic acid used during the preparation of the nanoparticles. In other embodiments, disclosed nanoparticles may comprise the hydrophobic acid. For instance, in some embodiments, the acid content in disclosed nanoparticles may be between about 0.05 weight percent to about 30 weight percent, in some embodiments between about 0.5 weight percent to about 30 weight percent, in some embodiments between about 1 weight percent to about 30 weight percent, in some embodiments between about 2 weight percent to about 30 weight percent, in some embodiments between about 3 weight percent to about 30 weight percent, in some embodiments between about 5 weight percent to about 30 weight percent, in some embodiments between about 7 weight percent to about 30 weight percent, in some embodiments between about 10 weight percent to about 30 weight percent, in some embodiments between about 15 weight percent to about 30 weight percent, in some embodiments between about 20 weight percent to about 30 weight percent, in some embodiments between about 0.05 weight percent to about 0.5 weight percent, in some embodiments between about 0.05 weight percent to about 5 weight percent, in some embodiments between about 1 weight percent to about 5 weight percent, in some embodiments between about 3 weight percent to about 10 weight percent, in some embodiments between about 5 weight percent to about 15 weight percent, and in some embodiments between about 10 weight percent to about 20 weight percent.

In some embodiments, disclosed nanoparticles substantially immediately release (*e.g*., over about 1 minute to about 30 minutes, about 1 minute to about 25 minutes, about 5 minutes to about 30 minutes, about 5 minutes to about 1 hour, about 1 hour, or about 24 hours) less than about 2%, less than about 5%, less than about 10%, less than about 15%, less than about 20%, less than about 25%, less than about 30%, or less than 40% of the therapeutic agent, for example when placed in a phosphate buffer solution at room temperature (*e*.*g*., 25°C) and/or at 37 °C.

In certain embodiments, nanoparticles comprising the therapeutic agent may release the therapeutic agent when placed in an aqueous solution (*e.g*., a phosphate buffer solution), *e.g*., at 25 °C and/or at 37°C, at a rate substantially corresponding to about 0.01 to about 50%, in some embodiments about 0.01 to about 25%, in some embodiments about 0.01 to about 15%, in some embodiments about 0.01 to about 10%, in some embodiments about 1 to about 40%, in some embodiments about 5 to about 40%, and in some embodiments about 10 to about 40% of the therapeutic agent released over about 1 hour.

In some embodiments, nanoparticles comprising the therapeutic agent may release the therapeutic agent when placed in an aqueous solution (*e.g*., a phosphate buffer solution), *e.g.*, at 25 °C and/or at 37°C, at a rate substantially corresponding to about 10 to about 70%, in some embodiments about 10 to about 45%, in some embodiments about 10 to about 35%, or in some embodiments about 10 to about 25%, of the therapeutic agent released over about 4 hours.

In certain embodiments, nanoparticles comprising the therapeutic agent may release the therapeutic agent when placed in an aqueous solution (*e.g.*, a phosphate buffer solution), *e.g.*, at 25 °C and/or at 37 °C, at a rate substantially corresponding to about 0.01 to about 60%, in some embodiments about 0.01 to about 50%, in some embodiments about 0.01 to about 45%, in some embodiments about 0.01 to about 40%, in some embodiments about 0.01 to about 35%, in some embodiments about 0.01 to about 30%, in some embodiments about 0.01 to about 25%, in some embodiments about 0.01 to about 15%, in some embodiments about 0.01 to about 10%, in some embodiments about 1 to about 50%, in some embodiments about 5 to about 50%, in some embodiments about 10 to about 50%, in some embodiments about 20 to about 50%, in some embodiments about 10 to about 30%, and in some embodiments about 1 to about 10%, of the therapeutic agent released over about 24 hours.

In some embodiments, nanoparticles comprising the therapeutic agent may release the therapeutic agent when placed in an aqueous solution (*e.g.*, a phosphate buffer solution), *e.g.*, at 25 °C and/or at 37 °C, at a rate substantially corresponding to about 1 to about 70%, in some embodiments about 2 to about 70%, in some embodiments about 5 to about 70%, in some embodiments about 10 to about 70%, in some embodiments about 20 to about 70%, in some embodiments about 30 to about 70%, in some embodiments about 1 to about 60%, in some embodiments about 1 to about 50%, in some embodiments about 1 to about 40%, in some embodiments about 1 to about 30%, in some embodiments about 1 to about 20%, in some embodiments about 1 to about 10%, in some embodiments about 5 to about 35%, or in some embodiments about 25 to about 60%, of the therapeutic agent released over about 50 hours.

In some embodiments, disclosed nanoparticles may substantially retain the therapeutic agent, *e.g.*, for at least about 1 minute, at least about 1 hour, or more, when placed in a phosphate buffer solution at 37 °C.

In general, a "nanoparticle" refers to any particle having a diameter of less than 1000 nm, *e.g.*, about 10 nm to about 200 nm. Disclosed therapeutic nanoparticles may include nanoparticles having a diameter of about 60 to about 120 nm, or about 70 to about 120 nm, or about 80 to about 120 nm, or about 90 to about 120 nm, or about 100 to about 120 nm, or about 60 to about 130 nm, or about 70 to about 130 nm, or about 80 to about 130 nm, or about 90 to about 130 nm, or about 100 to about 130 nm, or about 110 to about 130 nm, or about 60 to about 140 nm, or about 70 to about 140 nm, or about 80 to about 140 nm, or about 90 to about 140 nm, or about 100 to about 140 nm, or about 110 to about 140 nm, or about 60 to about 150 nm, or about 70 to about 150 nm, or about 80 to about 150 nm, or about 90 to about 150 nm, or about 100 to about 150 nm, or about 110 to about 150 nm, or about 120 to about 150 nm.

### Polymers

In some embodiments, the nanoparticles may comprise a matrix of polymers and a therapeutic agent. In some embodiments, a therapeutic agent can be associated with at least part of the polymeric matrix. The therapeutic agent can be associated with the surface of, encapsulated within, surrounded by, and/or dispersed throughout the polymeric matrix.

Any suitable polymer can be used in the disclosed nanoparticles. Polymers can be natural or unnatural (synthetic) polymers. Polymers can be homopolymers or copolymers comprising two or more monomers. In terms of sequence, copolymers can be random, block, or comprise a combination of random and block sequences. Typically, polymers are organic polymers.

The term "polymer," as used herein, is given its ordinary meaning as used in the art, *i.e.*, a molecular structure comprising one or more repeat units (monomers), connected by covalent bonds. The repeat units may all be identical, or in some cases, there may be more than one type of repeat unit present within the polymer. In some cases, the polymer can be biologically derived, *i.e.*, a biopolymer. Non-limiting examples include peptides or proteins. In some cases, additional moieties may also be present in the polymer, for example biological moieties such as those described below. If more than one type of repeat unit is present within the polymer, then the polymer is said to be a "copolymer." It is to be understood that in any embodiment employing a polymer, the polymer being employed may be a copolymer in some cases. The repeat units forming the copolymer may be arranged in any fashion. For example, the repeat units may be arranged in a random order, in an alternating order, or as a block copolymer, *i.e.*, comprising one or more regions each comprising a first repeat unit (*e.g*., a first block), and one or more regions each comprising a second repeat unit (*e.g.*, a second block), *etc.* Block copolymers may have two (a diblock copolymer), three (a triblock copolymer), or more numbers of distinct blocks.

Disclosed particles can include copolymers, which, in some embodiments, describes two or more polymers (such as those described herein) that have been associated with each other, usually by covalent bonding of the two or more polymers together. Thus, a copolymer may comprise a first polymer and a second polymer, which have been conjugated together to form a block copolymer where the first polymer can be a first block of the block copolymer and the second polymer can be a second block of the block copolymer. Of course, those of ordinary skill in the art will understand that a block copolymer may, in some cases, contain multiple blocks of polymer, and that a "block copolymer," as used herein, is not limited to only block copolymers having only a single first block and a single second block. For instance, a block copolymer may comprise a first block comprising a first polymer, a second block comprising a second polymer, and a third block comprising a third polymer or the first polymer, *etc.* In some cases, block copolymers can contain any number of first blocks of a first polymer and second blocks of a second polymer (and in certain cases, third blocks, fourth blocks, *etc.*). In addition, it should be noted that block copolymers can also be formed, in some instances, from other block copolymers. For example, a first block copolymer may be conjugated to another polymer (which may be a homopolymer, a biopolymer, another block copolymer, *etc.*), to form a new block copolymer containing multiple types of blocks, and/or to other moieties (*e*.*g*., to non-polymeric moieties).

In some embodiments, the polymer (*e.g.*, copolymer, *e.g.*, block copolymer) can be amphiphilic, *i.e.*, having a hydrophilic portion and a hydrophobic portion, or a relatively hydrophilic portion and a relatively hydrophobic portion. A hydrophilic polymer can be one generally that attracts water and a hydrophobic polymer can be one that generally repels water. A hydrophilic or a hydrophobic polymer can be identified, for example, by preparing a sample of the polymer and measuring its contact angle with water (typically, the polymer will have a contact angle of less than 60°, while a hydrophobic polymer will have a contact angle of greater than about 60°). In some cases, the hydrophilicity of two or more polymers may be measured relative to each other, *i.e.*, a first polymer may be more hydrophilic than a second polymer. For instance, the first polymer may have a smaller contact angle than the second polymer.

In one set of embodiments, a polymer (*e.g.*, copolymer, *e.g.*, block copolymer) contemplated herein includes a biocompatible polymer, *i.e.*, the polymer that does not typically induce an adverse response when inserted or injected into a living subject, for example, without significant inflammation and/or acute rejection of the polymer by the immune system, for instance, *via* a T-cell response. Accordingly, the therapeutic particles contemplated herein can be non-immunogenic. The term non-immunogenic as used herein refers to endogenous growth factor in its native state which normally elicits no, or only minimal levels of, circulating antibodies, T-cells, or reactive immune cells, and which normally does not elicit in the individual an immune response against itself.

Biocompatibility typically refers to the acute rejection of material by at least a portion of the immune system, *i.e.*, a nonbiocompatible material implanted into a subject provokes an immune response in the subject that can be severe enough such that the rejection of the material by the immune system cannot be adequately controlled, and often is of a degree such that the material must be removed from the subject. One simple test to determine biocompatibility can be to expose a polymer to cells *in vitro;* biocompatible polymers are polymers that typically will not result in significant cell death at moderate concentrations, *e*.*g*., at concentrations of 50 micrograms/10⁶ cells. For instance, a biocompatible polymer may cause less than about 20% cell death when exposed to cells such as fibroblasts or epithelial cells, even if phagocytosed or otherwise uptaken by such cells. Non-limiting examples of biocompatible polymers that may be useful in various embodiments include polydioxanone (PDO), polyhydroxyalkanoate, polyhydroxybutyrate, poly(glycerol sebacate), polyglycolide (*i.e*., poly(glycolic) acid) (PGA), polylactide (*i.e*., poly(lactic) acid) (PLA), poly(lactic) acid-co-poly(glycolic) acid (PLGA), polycaprolactone, or copolymers or derivatives including these and/or other polymers.

In certain embodiments, contemplated biocompatible polymers may be biodegradable, *i.e*., the polymer is able to degrade, chemically and/or biologically, within a physiological environment, such as within the body. As used herein, "biodegradable" polymers are those that, when introduced into cells, are broken down by the cellular machinery (biologically degradable) and/or by a chemical process, such as hydrolysis, (chemically degradable) into components that the cells can either reuse or dispose of without significant toxic effect on the cells. In one embodiment, the biodegradable polymer and their degradation byproducts can be biocompatible.

Particles disclosed herein may or may not contain PEG. In addition, certain embodiments can be directed towards copolymers containing poly(ester-ether)s, *e*.*g*.*,* polymers having repeat units joined by ester bonds (*e.g.*, R-C(O)-O-R' bonds) and ether bonds (*e.g.*, R-OR' bonds). In some embodiments, a biodegradable polymer, such as a hydrolyzable polymer, containing carboxylic acid groups, may be conjugated with poly(ethylene glycol) repeat units to form a poly(ester-ether). A polymer (*e.g.*, copolymer, *e.g.*, block copolymer) containing poly(ethylene glycol) repeat units can also be referred to as a "PEGylated" polymer.

For instance, a contemplated polymer may be one that hydrolyzes spontaneously upon exposure to water (e.g., within a subject), or the polymer may degrade upon exposure to heat (*e.g.*, at temperatures of about 37°C). Degradation of a polymer may occur at varying rates, depending on the polymer or copolymer used. For example, the half-life of the polymer (the time at which 50% of the polymer can be degraded into monomers and/or other nonpolymeric moieties) may be on the order of days, weeks, months, or years, depending on the polymer. The polymers may be biologically degraded, *e.g.*, by enzymatic activity or cellular machinery, in some cases, for example, through exposure to a lysozyme (*e*.*g*., having relatively low pH). In some cases, the polymers may be broken down into monomers and/or other nonpolymeric moieties that cells can either reuse or dispose of without significant toxic effect on the cells (for example, polylactide may be hydrolyzed to form lactic acid, polyglycolide may be hydrolyzed to form glycolic acid, *etc.*).

In some embodiments, polymers may be polyesters, including copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly-L-lactic acid, poly-D-lactic acid, poly-D,L-lactic acid, poly-L-lactide, poly-D-lactide, and poly-D,L-lactide, collectively referred to herein as "PLA." In some embodiments, exemplary polyesters include, for example, polyhydroxyacids; PEGylated polymers and copolymers of lactide and glycolide (*e*.*g*., PEGylated PLA, PEGylated PGA, PEGylated PLGA, and derivatives thereof). In some embodiments, polyesters include, for example, polyanhydrides, poly(ortho ester) PEGylated poly(ortho ester), poly(caprolactone), PEGylated poly(caprolactone), polylysine, PEGylated polylysine, poly(ethylene imine), PEGylated poly(ethylene imine), poly(L-lactide-co-L-lysine), poly(serine ester), poly(4-hydroxy-L-proline ester), poly[α-(4-aminobutyl)-L-glycolic acid], and derivatives thereof.

In some embodiments, a polymer may be PLGA. PLGA is a biocompatible and biodegradable co-polymer of lactic acid and glycolic acid, and various forms of PLGA can be characterized by the ratio of lactic acid:glycolic acid. Lactic acid can be L-lactic acid, D-lactic acid, or D,L-lactic acid. The degradation rate of PLGA can be adjusted by altering the lactic acid-glycolic acid ratio. In some embodiments, PLGA can be characterized by a lactic acid:glycolic acid ratio of approximately 85:15, approximately 75:25, approximately 60:40, approximately 50:50, approximately 40:60, approximately 25:75, or approximately 15:85. In some embodiments, the ratio of lactic acid to glycolic acid monomers in the polymer of the particle (*e.g.*, the PLGA block copolymer or PLGA-PEG block copolymer), may be selected to optimize for various parameters such as water uptake, therapeutic agent release and/or polymer degradation kinetics can be optimized.

In some embodiments, polymers may be one or more acrylic polymers. In certain embodiments, acrylic polymers include, for example, acrylic acid and methacrylic acid copolymers, methyl methacrylate copolymers, ethoxyethyl methacrylates, cyanoethyl methacrylate, amino alkyl methacrylate copolymer, poly(acrylic acid), poly(methacrylic acid), methacrylic acid alkylamide copolymer, poly(methyl methacrylate), poly(methacrylic acid polyacrylamide, amino alkyl methacrylate copolymer, glycidyl methacrylate copolymers, polycyanoacrylates, and combinations comprising one or more of the foregoing polymers. The acrylic polymer may comprise fully-polymerized copolymers of acrylic and methacrylic acid esters with a low content of quaternary ammonium groups.

In some embodiments, polymers can be cationic polymers. In general, cationic polymers are able to condense and/or protect negatively charged strands of nucleic acids *(e.g.,* DNA, RNA, or derivatives thereof). Amine-containing polymers such as poly(lysine), polyethylene imine (PEI), and poly(amidoamine) dendrimers are contemplated for use, in some embodiments, in a disclosed particle.

In some embodiments, polymers can be degradable polyesters bearing cationic side chains. Examples of these polyesters include poly(L-lactide-co-L-lysine), poly(serine ester), poly(4-hydroxy-L-proline ester).

It is contemplated that PEG may be terminated and include an end group. For example, PEG may terminate in a hydroxyl, a methoxy or other alkoxyl group, a methyl or other alkyl group, an aryl group, a carboxylic acid, an amine, an amide, an acetyl group, a guanidino group, or an imidazole. Other contemplated end groups include azide, alkyne, maleimide, aldehyde, hydrazide, hydroxylamine, alkoxyamine, or thiol moieties.

Those of ordinary skill in the art will know of methods and techniques for PEGylating a polymer, for example, by using EDC (l-ethyl-3-(3-dimethylaminopropyl) carbodiimide hydrochloride) and NHS (N-hydroxysuccinimide) to react a polymer to a PEG group terminating in an amine, by ring opening polymerization techniques (ROMP), or the like.

In one embodiment, the molecular weight (or *e.g.*, the ratio of molecular weights of, *e.g.*, different blocks of a copolymer) of the polymers can be optimized for effective treatment as disclosed herein. For example, the molecular weight of a polymer may influence particle degradation rate (such as when the molecular weight of a biodegradable polymer can be adjusted), solubility, water uptake, and drug release kinetics. For example, the molecular weight of the polymer (or *e.g.*, the ratio of molecular weights of, *e.g.*, different blocks of a copolymer) can be adjusted such that the particle biodegrades in the subject being treated within a reasonable period of time (ranging from a few hours to 1-2 weeks, 3-4 weeks, 5-6 weeks, 7-8 weeks, *etc.*).

A disclosed particle can for example comprise a diblock copolymer of PEG and PL(G)A, wherein for example, the PEG portion may have a number average molecular weight of about 1,000-20,000, *e.g*., about 2,000-20,000, *e.g*., about 2 to about 10,000, and the PL(G)A portion may have a number average molecular weight of about 5,000 to about 20,000, or about 5,000-100,000, *e.g*., about 20,000-70,000, *e.g*., about 15,000-50,000.

For example, disclosed here is an exemplary therapeutic nanoparticle that includes about 10 to about 99 weight percent poly(lactic) acid-poly(ethylene)glycol copolymer or poly(lactic)-co-poly (glycolic) acid-poly(ethylene)glycol copolymer, or about 20 to about 80 weight percent, about 40 to about 80 weight percent, or about 30 to about 50 weight percent, or about 70 to about 90 weight percent poly(lactic) acid-poly(ethylene)glycol copolymer or poly(lactic)-co-poly (glycolic) acid-poly(ethylene)glycol copolymer. Exemplary poly(lactic) acid-poly(ethylene)glycol copolymers can include a number average molecular weight of about 15 to about 20 kDa, or about 10 to about 25 kDa of poly(lactic) acid and a number average molecular weight of about 4 to about 6, or about 2kDa to about 10 kDa of poly(ethylene)glycol.

In some embodiments, the poly(lactic) acid-poly(ethylene)glycol copolymer may have a poly(lactic) acid number average molecular weight fraction of about 0.6 to about 0.95, in some embodiments between about 0.7 to about 0.9, in some embodiments between about 0.6 to about 0.8, in some embodiments between about 0.7 to about 0.8, in some embodiments between about 0.75 to about 0.85, in some embodiments between about 0.8 to about 0.9, and in some embodiments between about 0.85 to about 0.95. It should be understood that the poly(lactic) acid number average molecular weight fraction may be calculated by dividing the number average molecular weight of the poly(lactic) acid component of the copolymer by the sum of the number average molecular weight of the poly(lactic) acid component and the number average molecular weight of the poly(ethylene)glycol component.

Disclosed nanoparticles may optionally include about 1 to about 50 weight percent poly(lactic) acid or poly(lactic) acid-co-poly (glycolic) acid (which does not include PEG), or may optionally include about 1 to about 50 weight percent, or about 10 to about 50 weight percent or about 30 to about 50 weight percent poly(lactic) acid or poly(lactic) acid-co-poly (glycolic) acid. For example, poly(lactic) or poly(lactic)-co-poly(glycolic) acid may have a number average molecule weight of about 5 to about 15 kDa, or about 5 to about 12 kDa. Exemplary PLA may have a number average molecular weight of about 5 to about 10 kDa. Exemplary PLGA may have a number average molecular weight of about 8 to about 12 kDa.

A therapeutic nanoparticle may, in some embodiments, contain about 10 to about 30 weight percent, in some embodiments about 10 to about 25 weight percent, in some embodiments about 10 to about 20 weight percent, in some embodiments about 10 to about 15 weight percent, in some embodiments about 15 to about 20 weight percent, in some embodiments about 15 to about 25 weight percent, in some embodiments about 20 to about 25 weight percent, in some embodiments about 20 to about 30 weight percent, or in some embodiments about 25 to about 30 weight percent of poly(ethylene)glycol, where the poly(ethylene)glycol may be present as a poly(lactic) acid-poly(ethylene)glycol copolymer, poly(lactic)-co-poly (glycolic) acid-poly(ethylene)glycol copolymer, or poly(ethylene)glycol homopolymer. In certain embodiments, the polymers of the nanoparticles can be conjugated to a lipid. The polymer can be, for example, a lipid-terminated PEG.

### Preparation of Nanoparticles

Another aspect of this disclosure is directed to systems and methods of making disclosed nanoparticles. In some embodiments, using two or more different polymers (*e*.*g*., copolymers, *e*.*g*., block copolymers) in different ratios and producing particles from the polymers (*e.g*., copolymers, *e.g.*, block copolymers), properties of the particles be controlled. For example, a polymer (*e.g*., copolymer, *e.g*., block copolymer) may be chosen for its biocompatibility and/or its ability to control immunogenicity of the resultant particle.

In some embodiments, a solvent used in a nanoparticle preparation process (e.g., a nanoprecipitation process or a nanoemulsion process as discussed below) may include a hydrophobic acid, which may confer advantageous properties to the nanoparticles prepared using the process. As discussed above, in some cases, the hydrophobic acid may improve drug loading of disclosed nanoparticles. Furthermore, in some instances, the controlled release properties of disclosed nanoparticles may be improved by the use of the hydrophobic acid. In some cases, the hydrophobic acid may be included in, for example, an organic solution or an aqueous solution used in the process. In one embodiment, the drug is combined with an organic solution and the hydrophobic acid and optionally one or more polymers. The hydrophobic acid concentration in a solution used to dissolve the drug is discussed above and may be, for example, between about 1 weight percent and about 30 weight percent, etc.

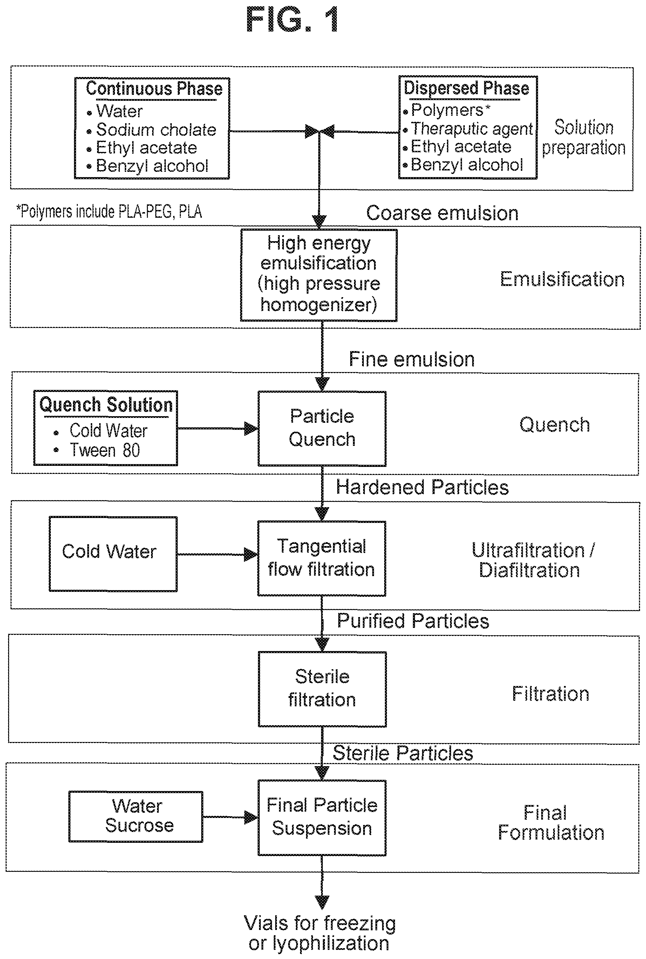

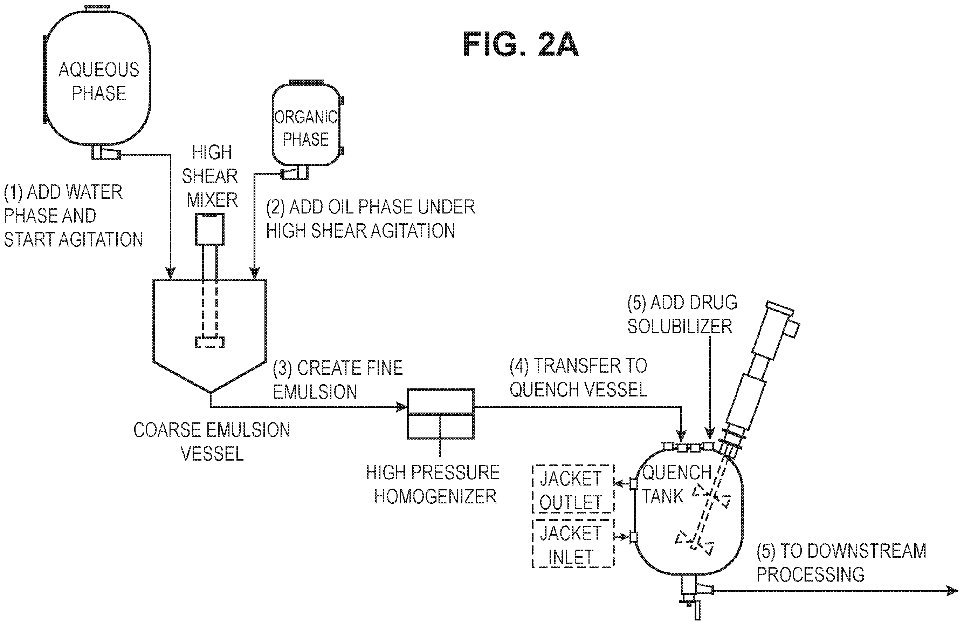

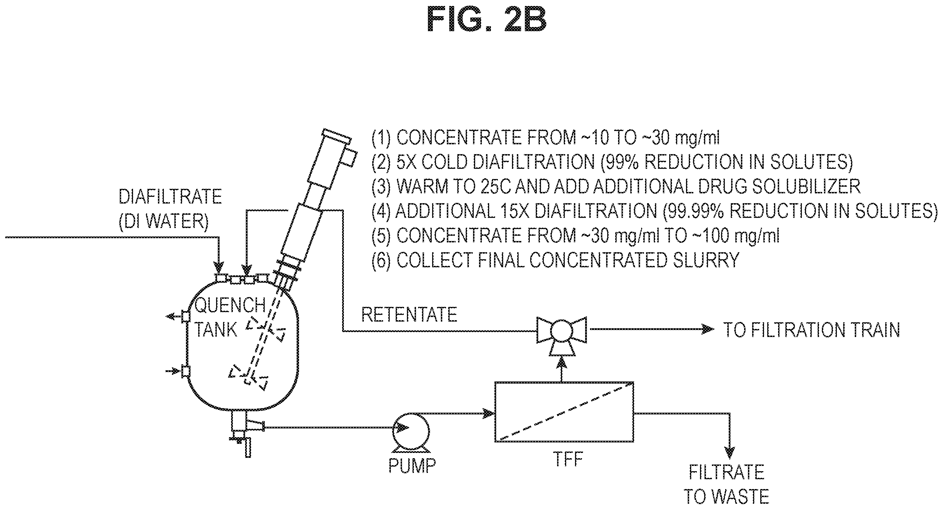

In an embodiment, a nanoemulsion process is provided, such as the process represented in FIGs. 1, 2A, and 2B. For example, the therapeutic agent, optionally a hydrophobic acid, a first polymer (for example, a diblock co-polymer such as PLA-PEG or PLGA-PEG) and an optional second polymer (*e*.*g*., (PL(G)A-PEG or PLA), may be combined with an organic solution to form a first organic phase. Such first phase may include about 1 to about 50% weight solids, about 5 to about 50% weight solids, about 5 to about 40% weight solids, about 1 to about 15% weight solids, or about 10 to about 30% weight solids. The first organic phase may be combined with a first aqueous phase, optionally containing a hydrophobic acid, to form a second phase. The organic solution can include, for example, toluene, methyl ethyl ketone, acetonitrile, tetrahydrofuran, ethyl acetate, isopropyl alcohol, isopropyl acetate, dimethylformamide, methylene chloride, dichloromethane, chloroform, acetone, benzyl alcohol, Tween 80, Span 80, or the like, and combinations thereof. In an embodiment, the organic phase may include benzyl alcohol, ethyl acetate, and combinations thereof. The second phase can be between about 0.1 and 50 weight %, between about 1 and 50 weight %, between about 5 and 40 weight %, or between about 1 and 15 weight %, solids. The aqueous phase can be water, optionally in combination with one or more of sodium cholate, ethyl acetate, polyvinyl acetate and benzyl alcohol.

In some embodiments, the pH of the aqueous phase may be selected based on the pKₐ of the protonated therapeutic agent and/or the pKₐ of the hydrophobic acid. For example, in certain embodiments, the therapeutic agent, when protonated, may have a first pKₐ, the hydrophobic acid may have a second pKₐ, and the aqueous phase may have a pH equal to a pKₐ unit between the first pKₐ and the second pKₐ. In a particular embodiment, the pH of the aqueous phase may be equal to a pKₐ unit that is about equidistant between the first pKₐ and the second pKₐ.

For example, the oil or organic phase may use a solvent that is only partially miscible with the nonsolvent (water). Therefore, when mixed at a low enough ratio and/or when using water pre-saturated with the organic solvents, the oil phase remains liquid. The oil phase may be emulsified into an aqueous phase and, as liquid droplets, sheared into nanoparticles using, for example, high energy dispersion systems, such as homogenizers or sonicators. The aqueous portion of the emulsion, otherwise known as the "water phase" or "aqueous phase", may be a surfactant solution consisting of sodium cholate and pre-saturated with ethyl acetate and benzyl alcohol. In some instances, the organic phase (*e.g*., first organic phase) may include the therapeutic agent. Additionally, in certain embodiments, the aqueous phase (*e.g*., first aqueous phase) may include the substantially hydrophobic acid. In other embodiments, both the therapeutic agent and the substantially hydrophobic acid may be dissolved in the organic phase.

Emulsifying the second phase to form an emulsion phase may be performed, for example, in one or two emulsification steps. For example, a primary emulsion may be prepared, and then emulsified to form a fine emulsion. The primary emulsion can be formed, for example, using simple mixing, a high pressure homogenizer, probe sonicator, stir bar, or a rotor stator homogenizer. The primary emulsion may be formed into a fine emulsion through the use of *e.g.*, probe sonicator or a high pressure homogenizer, *e*.*g*., by using 1, 2, 3, or more passes through a homogenizer. For example, when a high pressure homogenizer is used, the pressure used may be about 30 to about 60 psi, about 40 to about 50 psi, about 1000 to about 8000 psi, about 2000 to about 4000 psi, about 4000 to about 8000 psi, or about 4000 to about 5000 psi, *e.g.,* about 2000, 2500, 4000 or 5000 psi.

In some cases, fine emulsion conditions, which can be characterized by a very high surface to volume ratio of the droplets in the emulsion, can be chosen to maximize the solubility of the therapeutic agent and hydrophobic acid and form the desired HIP. In certain embodiments, under fine emulsion conditions, equilibration of dissolved components can occur very quickly, *i.e.*, faster than solidification of the nanoparticles. Thus, selecting a HIP based on, *e.g.*, the pKₐ difference between the therapeutic agent and the hydrophobic acid, or adjusting other parameters such as the pH of the fine emulsion and/or the pH of the quench solution, can have a significant impact on the drug loading and release properties of the nanoparticles by dictating, for example, the formation of a HIP in the nanoparticle as opposed to diffusion of the therapeutic agent and/or hydrophobic acid out of the nanoparticle.

In some embodiments, the therapeutic agent and the substantially hydrophobic acid may be combined in the second phase prior to emulsifying the second phase. In some instances, the therapeutic agent and the substantially hydrophobic acid may form a hydrophobic ion pair prior to emulsifying the second phase. In other embodiments, the therapeutic agent and the substantially hydrophobic acid may form a hydrophobic ion pair during emulsification of the second phase. For example, the therapeutic agent and the substantially hydrophobic acid may be combined in the second phase substantially concurrently with emulsifying the second phase, *e.g.*, the therapeutic agent and the substantially hydrophobic acid may be dissolved in separate solutions (*e*.*g*., two substantially immiscible solutions), which are then combined during emulsification. In another example, the therapeutic agent and the substantially hydrophobic acid may be dissolved in separate miscible solutions that are then fed into second phase during emulsification.

Either solvent evaporation or dilution may be needed to complete the extraction of the solvent and solidify the particles. For better control over the kinetics of extraction and a more scalable process, a solvent dilution via aqueous quench may be used. For example, the emulsion can be diluted into cold water to a concentration sufficient to dissolve all of the organic solvent to form a quenched phase. In some embodiments, quenching may be performed at least partially at a temperature of about 5 °C or less. For example, water used in the quenching may be at a temperature that is less than room temperature (*e*.*g*., about 0 to about 10°C, or about 0 to about 5 °C). In certain embodiments, the quench may be chosen having a pH that is advantageous for quenching the emulsion phase, *e.g.*, by improving the properties of the nanoparticles, such as the release profile, or improving a nanoparticle parameter, such as the drug loading. The pH of the quench may be adjusted by acid or base titration, for example, or by appropriate selection of a buffer. In some embodiments, the pH of the quench may be selected based on the pKₐ of the protonated therapeutic agent and/or the pKₐ of the hydrophobic acid. For example, in certain embodiments, the therapeutic agent, when protonated, may have a first pKₐ, the hydrophobic acid may have a second pKₐ, and the emulsion phase may be quenched with an aqueous solution having a pH equal to a pKₐ unit between the first pKₐ and the second pKₐ. In some embodiments, the resultant quenched phase may also have a pH equal to a pKₐ unit between the first pKₐ and the second pKₐ. In a particular embodiment, the pH may be equal to a pKₐ unit that is about equidistant between the first pKₐ and the second pKₐ.

In certain embodiments, HIP formation can occur during or after emulsification, *e.g.,* as a result of equilibrium conditions in the fine emulsion. Without wishing to be bound by any theory, it is believed that organic-soluble counter ions (*i.e*., the hydrophobic acid) can facilitate diffusion of the therapeutic agent into a nanoparticle of an emulsion as a result of HIP formation. Without wishing to be bound by any theory, the HIP may remain in the nanoparticle before solidification of the nanoparticle since the solubility of the HIP in the nanoparticle is higher than the solubility of the HIP in the aqueous phase of the emulsion and/or in the quench. For example, by selecting a pH for the quench that is between the pKₐ of the therapeutic agent and the pKₐ of the hydrophobic acid, formation of ionized therapeutic agent and hydrophobic acid can be optimized. However, selecting a pH that is too high may tend to cause the hydrophobic acid to diffuse out of the nanoparticle, whereas selecting a pH that is too low may tend to cause the therapeutic agent to diffuse out of the nanoparticle.

In some embodiments, the quench may comprise a substantially hydrophobic acid having at least some water solubility. For example, in some embodiments, the substantially hydrophobic acid having at least some water solubility may contain between 6 and 16 carbon atoms, in some embodiments between 6 and 12 carbon atoms, in some embodiments between 6 and 10 carbon atoms, in some embodiments between 8 and 16 carbon atoms, in some embodiments between 10 and 16 carbon atoms, or in some embodiments between 12 and 16 carbon atoms. Non-limiting examples of substantially hydrophobic acids having at least some water solubility include caproic acid (*i.e.*, hexanoic acid), enanthic acid (*i.e*., heptanoic acid), caprylic acid (*i.e.*, octanoic acid), pelargonic acid (*i.e.*, nonanoic acid), capric acid (*i.e*., decanoic acid), undecanoic acid, lauric acid (*i.e.*, dodecanoic acid), tridecanoic acid, myristic acid (*i.e.,* tetradecanoic acid), pentadecanoic acid, palmitic acid (*i.e*., hexadecanoic acid), cinnamic acid, phenylacetic acid, dodecylbenzenesulfonic acid, dioctyl sulfosuccinic acid, dioleoyl phosphatidic acid, bile acids (*e*.*g*., chenodeoxycholic acid, ursodeoxycholic acid, deoxycholic acid, cholic acid, and lithocholic acid), and combinations thereof.

In some embodiments, the substantially hydrophobic acid having at least some water solubility may be a mixture of two or more substantially hydrophobic acids.

In some embodiments, the substantially hydrophobic acid in the quench may be in addition to a substantially hydrophobic acid in the aqueous phase and/or organic phase used to generate the emulsion phase. In other embodiments, the quench may comprise a substantially hydrophobic acid and the aqueous phase and/or organic phase used to generate the emulsion phase may be essentially free of a substantially hydrophobic acid. In certain embodiments, the substantially hydrophobic acid in the quench may be the same as a substantially hydrophobic acid used elsewhere in a contemplated formulation (e.g., in the aqueous phase and/or organic phase used to form an emulsion). In certain embodiments, the substantially hydrophobic acid in the quench may be different than a substantially hydrophobic acid used elsewhere in a contemplated formulation (*e*.*g*., in the aqueous phase and/or organic phase used to form an emulsion).

In certain embodiments, quenching an emulsion phase using a quench comprising a substantially hydrophobic acid may improve the encapsulation efficiency for loading the contemplated nanoparticles with a therapeutic agent. For example, in some embodiments, the encapsulation efficiency may be between about 50% and about 100%, in some embodiments between about 75% and about 100%, in some embodiments between about 85% and about 100%, in some embodiments between about 90% and about 100%, and in some embodiments between about 95% and about 100%. In certain embodiments, the encapsulation efficiency may be about 100%. Advantageously, in some embodiments, nanoparticles prepared using a process comprising a quench that includes a substantially hydrophobic acid having at least some water solubility may have a higher drug loading in comparison to nanoparticles prepared using a quench that does not contain a substantially hydrophobic acid.

In some embodiments, including a substantially hydrophobic acid having at least some water solubility in a quench used to prepare the contemplated nanoparticles may affect the release properties of the contemplated nanoparticles. For example, in certain embodiments, the release rate of a therapeutic agent from the nanoparticles may be increased by when the molecular weight of the substantially hydrophobic acid in the quench is decreased.

In some embodiments, the pH of an aqueous solution used in a nanoparticle formulation process (*e*.*g*., including, but not limited to, the aqueous phase, the emulsion phase, the quench, and the quenched phase) may be independently selected and may be between about 1 and about 3, in some embodiments between about 2 and about 4, in some embodiments between about 3 and about 5, in some embodiments between about 4 and about 6, in some embodiments between about 5 and about 7, in some embodiments between about 6 and about 8, in some embodiments between about 7 and about 9, and in some embodiments between about 8 and about 10. In certain embodiments, the pH of an aqueous solution used in a nanoparticle formulation process may be between about 3 and about 4, in some embodiments between about 4 and about 5, in some embodiments between about 5 and about 6, in some embodiments between about 6 and about 7, in some embodiments between about 7 and about 8, and in some embodiments between about 8 and about 9.

In some embodiments, not all of the therapeutic agent is encapsulated in the particles at this stage, and a drug solubilizer is added to the quenched phase to form a solubilized phase. The drug solubilizer may be for example, Tween 80, Tween 20, polyvinyl pyrrolidone, cyclodextran, sodium dodecyl sulfate, sodium cholate, diethylnitrosamine, sodium acetate, urea, glycerin, propylene glycol, glycofurol, poly(ethylene)glycol, bis(polyoxyethyleneglycol)dodecyl ether, sodium benzoate, sodium salicylate, or combinations thereof. For example, Tween-80 may be added to the quenched nanoparticle suspension to solubilize the free drug and prevent the formation of drug crystals. In some embodiments, a ratio of drug solubilizer to the therapeutic agent is about 200:1 to about 10:1, or in some embodiments about 100:1 to about 10:1.

The solubilized phase may be filtered to recover the nanoparticles. For example, ultrafiltration membranes may be used to concentrate the nanoparticle suspension and substantially eliminate organic solvent, free drug (*i.e*., unencapsulated therapeutic agent), drug solubilizer, and other processing aids (surfactants). Exemplary filtration may be performed using a tangential flow filtration system. For example, by using a membrane with a pore size suitable to retain nanoparticles while allowing solutes, micelles, and organic solvent to pass, nanoparticles can be selectively separated. Exemplary membranes with molecular weight cutoffs of about 300-500 kDa (∼5-25 nm) may be used.

Diafiltration may be performed using a constant volume approach, meaning the diafiltrate (cold deionized water, *e*.*g*., about 0 to about 5°C, or 0 to about 10°C) may added to the feed suspension at the same rate as the filtrate is removed from the suspension. In some embodiments, filtering may include a first filtering using a first temperature of about 0 to about 5°C, or 0 to about 10°C, and a second temperature of about 20 to about 30 °C, or 15 to about 35°C. In some embodiments, filtering may include processing about 1 to about 30, in some cases about 1 to about 15, or in some cases 1 to about 6 diavolumes. For example, filtering may include processing about 1 to about 30, or in some cases about 1 to about 6 diavolumes, at about 0 to about 5°C, and processing at least one diavolume (*e*.*g*., about 1 to about 15, about 1 to about 3, or about 1 to about 2 diavolumes) at about 20 to about 30°C. In some embodiments, filtering comprises processing different diavolumes at different distinct temperatures.

After purifying and concentrating the nanoparticle suspension, the particles may be passed through one, two or more sterilizing and/or depth filters, for example, using ∼0.2 µm depth pre-filter. For example, a sterile filtration step may involve filtering the therapeutic nanoparticles using a filtration train at a controlled rate. In some embodiments, the filtration train may include a depth filter and a sterile filter.

In another embodiment of preparing nanoparticles, an organic phase is formed composed of a mixture of the therapeutic agent and polymer (homopolymer and co-polymer). The organic phase is mixed with an aqueous phase at approximately a 1:5 ratio (oil phase:aqueous phase) where the aqueous phase is composed of a surfactant and some dissolved solvent. The primary emulsion is formed by the combination of the two phases under simple mixing or through the use of a rotor stator homogenizer. The primary emulsion is then formed into a fine emulsion through the use of a high pressure homogenizer. The fine emulsion is then quenched by addition to deionized water under mixing. In some embodiments, the quench:emulsion ratio may be about 2:1 to about 40: 1, or in some embodiments about 5:1 to about 15:1. In some embodiments, the quench:emulsion ratio is approximately 8.5:1. Then a solution of Tween (e.g., Tween 80) is added to the quench to achieve approximately 2% Tween overall. This serves to dissolve free, unencapsulated therapeutic agent. The nanoparticles are then isolated through either centrifugation or ultrafiltration/diafiltration.

It will be appreciated that the amounts of polymer, therapeutic agent, and hydrophobic acid that are used in the preparation of the formulation may differ from a final formulation. For example, some of the therapeutic agent may not become completely incorporated in a nanoparticle and such free therapeutic agent may be *e.g.*, filtered away. For example, in an embodiment, a first organic solution containing about 11 weight percent theoretical loading of therapeutic agent in a first organic solution containing about 9% of a first hydrophobic acid (*e.g*., a fatty acid), a second organic solution containing about 89 weight percent polymer (*e*.*g*., the polymer may include PLA-PEG), and an aqueous solution containing about 0.12% of a second hydrophobic acid may be used in the preparation of a formulation that results in, *e.g.*, a final nanoparticle comprising about 2 weight percent therapeutic agent, about 97.5 weight percent polymer, and about 0.5% total hydrophobic acid. Such processes may provide final nanoparticles suitable for administration to a patient that includes about 1 to about 20 percent by weight therapeutic agent, *e*.*g*.*,* about 1, about 2, about 3, about 4, about 5, about 8, about 10, or about 15 percent therapeutic agent by weight.

### Therapeutic Agents

Contemplated nanoparticles may contain any suitable therapeutic agent. Suitable lists of known agents are well known to those of ordinary skill in the art and include, but are not limited to, the Merck Index and the FDA Orange Book, each of which is incorporated herein by reference.

Contemplated nanoparticles may be useful, for example, in embodiments where a targeting moiety may be used to direct a particle containing a drug to a particular localized location within a subject, *e.g.*, to allow localized delivery of the drug to occur. In one set of embodiments, a combination of more than one therapeutic agents may be used.

Non-limiting examples of therapeutic agents include chemotherapeutic agents such Bcr-Abl tyrosine-kinase inhibitors (e.g., imatinib, nilotinib, dasatinib, bosutinib, ponatinib, and bafetinib), doxorubicin (adriamycin), gemcitabine (gemzar), daunorubicin, procarbazine, mitomycin, cytarabine, etoposide, methotrexate, venorelbine, 5-fluorouracil (5-FU), vinca alkaloids such as vinblastine or vincristine; bleomycin, paclitaxel (taxol), docetaxel (taxotere), cabazitaxel, aldesleukin, asparaginase, busulfan, carboplatin, cladribine, camptothecin, CPT-11, 10-hydroxy-7-ethylcamptothecin (SN38), dacarbazine, S-I capecitabine, ftorafur, 5'deoxyflurouridine, UFT, eniluracil, deoxycytidine, 5-azacytosine, 5-azadeoxycytosine, allopurinol, 2-chloroadenosine, trimetrexate, aminopterin, methylene-10-deazaaminopterin (MDAM), oxaplatin, picoplatin, tetraplatin, satraplatin, platinum-DACH, ormaplatin, CI-973, JM-216, and analogs thereof, epirubicin, etoposide phosphate, 9- aminocamptothecin, 10,11-methylenedioxycamptothecin, karenitecin, 9-nitrocamptothecin, TAS 103, vindesine, L-phenylalanine mustard, ifosphamidemefosphamide, perfosfamide, trophosphamide carmustine, semustine, epothilones A-E, tomudex, 6-mercaptopurine, 6-thioguanine, amsacrine, etoposide phosphate, karenitecin, acyclovir, valacyclovir, ganciclovir, amantadine, rimantadine, lamivudine, zidovudine, bevacizumab, trastuzumab, rituximab, 5-Fluorouracil, and combinations thereof.

Non-limiting examples of potentially suitable combination drugs include anti-cancer agents, including, for example, cabazitaxel, mitoxantrone, and mitoxantrone hydrochloride. In another embodiment, the payload may be an anti-cancer drug such as 20-epi-l, 25 dihydroxyvitamin D3, 4-ipomeanol, 5-ethynyluracil, 9-dihydrotaxol, abiraterone, acivicin, aclarubicin, acodazole hydrochloride, acronine, acylfiilvene, adecypenol, adozelesin, aldesleukin, all-tk antagonists, altretamine, ambamustine, ambomycin, ametantrone acetate, amidox, amifostine, aminoglutethimide, aminolevulinic acid, amrubicin, amsacrine, anagrelide, anastrozole, andrographolide, angiogenesis inhibitors, antagonist D, antagonist G, antarelix, anthramycin, anti-dorsalizdng morphogenetic protein-1, antiestrogen, antineoplaston, antisense oligonucleotides, aphidicolin glycinate, apoptosis gene modulators, apoptosis regulators, apurinic acid, ARA-CDP-DL-PTBA, arginine deaminase, asparaginase, asperlin, asulacrine, atamestane, atrimustine, axinastatin 1, axinastatin 2, axinastatin 3, azacitidine, azasetron, azatoxin, azatyrosine, azetepa, azotomycin, baccatin III derivatives, balanol, batimastat, benzochlorins, benzodepa, benzoylstaurosporine, beta lactam derivatives, beta-alethine, betaclamycin B, betulinic acid, BFGF inhibitor, bicalutamide, bisantrene, bisantrene hydrochloride, bisazuidinylspermine, bisnafide, bisnafide dimesylate, bistratene A, bizelesin, bleomycin, bleomycin sulfate, BRC/ABL antagonists, breflate, brequinar sodium, bropirimine, budotitane, busulfan, buthionine sulfoximine, cactinomycin, calcipotriol, calphostin C, calusterone, camptothecin derivatives, canarypox IL-2, capecitabine, caraceraide, cabazitaxel, carbetimer, carboplatin, carboxamide-amino-triazole, carboxyamidotriazole, carest M3, carmustine, earn 700, cartilage derived inhibitor, carubicin hydrochloride, carzelesin, casein kinase inhibitors, castanosperrnine, cecropin B, cedefingol, cetrorelix, chlorambucil, chlorins, chloroquinoxaline sulfonamide, cicaprost, cirolemycin, cisplatin, cis-porphyrin, cladribine, clomifene analogs, clotrimazole, collismycin A, collismycin B, combretastatin A4, combretastatin analog, conagenin, crambescidin 816, crisnatol, crisnatol mesylate, cryptophycin 8, cryptophycin A derivatives, curacin A, cyclopentanthraquinones, cyclophosphamide, cycloplatam, cypemycin, cytarabine, cytarabine ocfosfate, cytolytic factor, cytostatin, dacarbazine, dacliximab, dactinomycin, daunorubicin hydrochloride, decitabine, dehydrodidemnin B, deslorelin, dexifosfamide, dexormaplatin, dexrazoxane, dexverapamil, dezaguanine, dezaguanine mesylate, diaziquone, didemnin B, didox, diethyhiorspermine, dihydro-5-azacytidine, dioxamycin, diphenyl spiromustine, docetaxel, docosanol, dolasetron, doxifluridine, doxorubicin, doxorubicin hydrochloride, droloxifene, droloxifene citrate, dromostanolone propionate, dronabinol, duazomycin, duocannycin SA, ebselen, ecomustine, edatrexate, edelfosine, edrecolomab, eflomithine, eflomithine hydrochloride, elemene, elsarnitrucin, emitefur, enloplatin, enpromate, epipropidine, epirubicin, epirubicin hydrochloride, epristeride, erbulozole, erythrocyte gene therapy vector system, esorubicin hydrochloride, estramustine, estramustine analog, estramustine phosphate sodium, estrogen agonists, estrogen antagonists, etanidazole, etoposide, etoposide phosphate, etoprine, exemestane, fadrozole, fadrozole hydrochloride, fazarabine, fenretinide, filgrastim, finasteride, flavopiridol, flezelastine, floxuridine, fluasterone, fludarabine, fludarabine phosphate, fluorodaunorunicin hydrochloride, fluorouracil, flurocitabine, forfenimex, formestane, fosquidone, fostriecin, fostriecin sodium, fotemustine, gadolinium texaphyrin, gallium nitrate, galocitabine, ganirelix, gelatinase inhibitors, gemcitabine, gemcitabine hydrochloride, glutathione inhibitors, hepsulfam, heregulin, hexamethylene bisacetamide, hydroxyurea, hypericin, ibandronic acid, idarubicin, idarubicin hydrochloride, idoxifene, idramantone, ifosfamide, ihnofosine, ilomastat, imidazoacridones, imiquimod, immunostimulant peptides, insulin-like growth factor-1 receptor inhibitor, interferon agonists, interferon alpha-2A, interferon alpha-2B, interferon alpha-Nl, interferon alpha-N3, interferon beta-IA, interferon gamma-IB, interferons, interleukins, iobenguane, iododoxorubicin, iproplatm, irinotecan, irinotecan hydrochloride, iroplact, irsogladine, isobengazole, isohomohalicondrin B, itasetron, jasplakinolide, kahalalide F, lamellarin-N triacetate, lanreotide, lanreotide acetate, leinamycin, lenograstim, lentinan sulfate, leptolstatin, letrozole, leukemia inhibiting factor, leukocyte alpha interferon, leuprolide acetate, leuprolide/estrogen/progesterone, leuprorelin, levamisole, liarozole, liarozole hydrochloride, linear polyamine analog, lipophilic disaccharide peptide, lipophilic platinum compounds, lissoclinamide, lobaplatin, lombricine, lometrexol, lometrexol sodium, lomustine, lonidamine, losoxantrone, losoxantrone hydrochloride, lovastatin, loxoribine, lurtotecan, lutetium texaphyrin lysofylline, lytic peptides, maitansine, mannostatin A, marimastat, masoprocol, maspin, matrilysin inhibitors, matrix metalloproteinase inhibitors, maytansine, mechlorethamine hydrochloride, megestrol acetate, melengestrol acetate, melphalan, menogaril, merbarone, mercaptopurine, meterelin, methioninase, methotrexate, methotrexate sodium, metoclopramide, metoprine, meturedepa, microalgal protein kinase C inhibitors, MIF inhibitor, mifepristone, miltefosine, mirimostim, mismatched double stranded RNA, mitindomide, mitocarcin, mitocromin, mitogillin, mitoguazone, mitolactol, mitomalcin, mitomycin, mitomycin analogs, mitonafide, mitosper, mitotane, mitotoxin fibroblast growth factor-saporin, mitoxantrone, mitoxantrone hydrochloride, mofarotene, molgramostim, monoclonal antibody, human chorionic gonadotrophin, monophosphoryl lipid a/myobacterium cell wall SK, mopidamol, multiple drug resistance gene inhibitor, multiple tumor suppressor 1-based therapy, mustard anticancer agent, mycaperoxide B, mycobacterial cell wall extract, mycophenolic acid, myriaporone, n-acetyldinaline, nafarelin, nagrestip, naloxone/pentazocine, napavin, naphterpin, nartograstim, nedaplatin, nemorubicin, neridronic acid, neutral endopeptidase, nilutamide, nisamycin, nitric oxide modulators, nitroxide antioxidant, nitrullyn, nocodazole, nogalamycin, n-substituted benzamides, O6-benzylguanine, octreotide, okicenone, oligonucleotides, onapristone, ondansetron, oracin, oral cytokine inducer, ormaplatin, osaterone, oxaliplatin, oxaunomycin, oxisuran, paclitaxel, paclitaxel analogs, paclitaxel derivatives, palauamine, palmitoylrhizoxin, pamidronic acid, panaxytriol, panomifene, parabactin, pazelliptine, pegaspargase, peldesine, peliomycin, pentamustine, pentosan polysulfate sodium, pentostatin, pentrozole, peplomycin sulfate, perflubron, perfosfamide, perillyl alcohol, phenazinomycin, phenylacetate, phosphatase inhibitors, picibanil, pilocarpine hydrochloride, pipobroman, piposulfan, pirarubicin, piritrexim, piroxantrone hydrochloride, placetin A, placetin B, plasminogen activator inhibitor, platinum complex, platinum compounds, platinum-triamine complex, plicamycin, plomestane, porfimer sodium, porfiromycin, prednimustine, procarbazine hydrochloride, propyl bis-acridone, prostaglandin J2, prostatic carcinoma antiandrogen, proteasome inhibitors, protein A-based immune modulator, protein kinase C inhibitor, protein tyrosine phosphatase inhibitors, purine nucleoside phosphorylase inhibitors, puromycin, puromycin hydrochloride, purpurins, pyrazorurin, pyrazoloacridine, pyridoxylated hemoglobin polyoxyethylene conjugate, RAF antagonists, raltitrexed, ramosetron, RAS farnesyl protein transferase inhibitors, RAS inhibitors, RAS-GAP inhibitor, retelliptine demethylated, rhenium RE 186 etidronate, rhizoxin, riboprine, ribozymes, RH retinarnide, RNAi, rogletimide, rohitukine, romurtide, roquinimex, rubiginone Bl, ruboxyl, safingol, safingol hydrochloride, saintopin, sarcnu, sarcophytol A, sargramostim, SDI1 mimetics, semustine, senescence derived inhibitor 1, sense oligonucleotides, signal transduction inhibitors, signal transduction modulators, simtrazene, single chain antigen binding protein, sizofiran, sobuzoxane, sodium borocaptate, sodium phenylacetate, solverol, somatomedin binding protein, sonermin, sparfosafe sodium, sparfosic acid, sparsomycin, spicamycin D, spirogermanium hydrochloride, spiromustine, spiroplatin, splenopentin, spongistatin 1, squalamine, stem cell inhibitor, stem-cell division inhibitors, stipiamide, streptonigrin, streptozocin, stromelysin inhibitors, sulfinosine, sulofenur, superactive vasoactive intestinal peptide antagonist, suradista, suramin, swainsonine, synthetic glycosaminoglycans, talisomycin, tallimustine, tamoxifen methiodide, tauromustine, tazarotene, tecogalan sodium, tegafur, tellurapyrylium, telomerase inhibitors, teloxantrone hydrochloride, temoporfin, temozolomide, teniposide, teroxirone, testolactone, tetrachlorodecaoxide, tetrazomine, thaliblastine, thalidomide, thiamiprine, thiocoraline, thioguanine, thiotepa, thrombopoietin, thrombopoietin mimetic, thymalfasin, thymopoietin receptor agonist, thymotrinan, thyroid stimulating hormone, tiazofurin, tin ethyl etiopurpurin, tirapazamine, titanocene dichloride, topotecan hydrochloride, topsentin, toremifene, toremifene citrate, totipotent stem cell factor, translation inhibitors, trestolone acetate, tretinoin, triacetyluridine, triciribine, triciribine phosphate, trimetrexate, trimetrexate glucuronate, triptorelin, tropisetron, tubulozole hydrochloride, turosteride, tyrosine kinase inhibitors, tyrphostins, UBC inhibitors, ubenimex, uracil mustard, uredepa, urogenital sinus-derived growth inhibitory factor, urokinase receptor antagonists, vapreotide, variolin B, velaresol, veramine, verdins, verteporfin, vinblastine sulfate, vincristine sulfate, vindesine, vindesine sulfate, vinepidine sulfate, vinglycinate sulfate, vinleurosine sulfate, vinorelbine or vinorelbine tartrate, vinrosidine sulfate, vinxaltine, vinzolidine sulfate, vitaxin, vorozole, zanoterone, zeniplatin, zilascorb, zinostatin, zinostatin stimalamer, or zorubicin hydrochloride.

### Pharmaceutical Formulations

Nanoparticles disclosed herein may be combined with pharmaceutically acceptable carriers to form a pharmaceutical composition, according to another aspect. As would be appreciated by one of skill in this art, the carriers may be chosen based on the route of administration as described below, the location of the target issue, the drug being delivered, the time course of delivery of the drug, *etc.*

The pharmaceutical compositions can be administered to a patient by any means known in the art including oral and parenteral routes. The term "patient," as used herein, refers to humans as well as non-humans, including, for example, mammals, birds, reptiles, amphibians, and fish. For instance, the non-humans may be mammals (*e*.*g*., a rodent, a mouse, a rat, a rabbit, a monkey, a dog, a cat, a primate, or a pig). In certain embodiments parenteral routes are desirable since they avoid contact with the digestive enzymes that are found in the alimentary canal. According to such embodiments, inventive compositions may be administered by injection (*e*.*g*., intravenous, subcutaneous or intramuscular, intraperitoneal injection), rectally, vaginally, topically (as by powders, creams, ointments, or drops), or by inhalation (as by sprays).

In a particular embodiment, the nanoparticles are administered to a subject in need thereof systemically, *e*.*g*., by IV infusion or injection.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension, or emulsion in a nontoxic parenterally acceptable diluent or solvent, for example, as a solution in 1,3-butanediol. Among the acceptable vehicles and solvents that may be employed are water, Ringer's solution, U.S.P., and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. In one embodiment, the inventive conjugate is suspended in a carrier fluid comprising 1 % (w/v) sodium carboxymethyl cellulose and 0.1% (v/v) TWEEN™ 80. The injectable formulations can be sterilized, for example, by filtration through a bacteria-retaining filter, or by incorporating sterilizing agents in the form of sterile solid compositions which can be dissolved or dispersed in sterile water or other sterile injectable medium prior to use.

Solid dosage forms for oral administration include capsules, tablets, pills, powders, and granules. In such solid dosage forms, the encapsulated or unencapsulated conjugate is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar-agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may also comprise buffering agents.

It will be appreciated that the exact dosage of a nanoparticle containing the therapeutic agent is chosen by the individual physician in view of the patient to be treated, in general, dosage and administration are adjusted to provide an effective amount of the therapeutic nanoparticle to the patient being treated. As used herein, the "effective amount" of a nanoparticle containing the therapeutic agent refers to the amount necessary to elicit the desired biological response. As will be appreciated by those of ordinary skill in this art, the effective amount of a nanoparticle containing the therapeutic agent may vary depending on such factors as the desired biological endpoint, the drug to be delivered, the target tissue, the route of administration, *etc.* For example, the effective amount of a nanoparticle containing the therapeutic agent might be the amount that results in a reduction in tumor size by a desired amount over a desired period of time. Additional factors which may be taken into account include the severity of the disease state; age, weight and gender of the patient being treated; diet, time and frequency of administration; drug combinations; reaction sensitivities; and tolerance/response to therapy.

The nanoparticles may be formulated in dosage unit form for ease of administration and uniformity of dosage. The expression "dosage unit form" as used herein refers to a physically discrete unit of nanoparticle appropriate for the patient to be treated. It will be understood, however, that the total daily usage of the compositions will be decided by the attending physician within the scope of sound medical judgment. For any nanoparticle, the therapeutically effective dose can be estimated initially either in cell culture assays or in animal models, usually mice, rabbits, dogs, or pigs. The animal model is also used to achieve a desirable concentration range and route of administration. Such information can then be used to determine useful doses and routes for administration in humans. Therapeutic efficacy and toxicity of nanoparticles can be determined by standard pharmaceutical procedures in cell cultures or experimental animals, e.g., ED₅₀ (the dose is therapeutically effective in 50% of the population) and LD₅₀ (the dose is lethal to 50% of the population). The dose ratio of toxic to therapeutic effects is the therapeutic index, and it can be expressed as the ratio, LD₅₀/ED₅₀. Pharmaceutical compositions which exhibit large therapeutic indices may be useful in some embodiments. The data obtained from cell culture assays and animal studies can be used in formulating a range of dosage for human use.

In an embodiment, compositions disclosed herein may include less than about 10 ppm of palladium, or less than about 8 ppm, or less than about 6 ppm of palladium. For example, provided here is a composition that includes nanoparticles wherein the composition has less than about 10 ppm of palladium.

In some embodiments, a composition suitable for freezing is contemplated, including nanoparticles disclosed herein and a solution suitable for freezing, *e.g*., a sugar such as a mono, di, or poly saccharide, *e*.*g*., sucrose and/or a trehalose, and/or a salt and/or a cyclodextrin solution is added to the nanoparticle suspension. The sugar (*e*.*g*., sucrose or trehalose) may act, *e.g.*, as a cryoprotectant to prevent the particles from aggregating upon freezing. For example, provided herein is a nanoparticle formulation comprising a plurality of disclosed nanoparticles, sucrose, an ionic halide, and water; wherein the nanoparticles/sucrose/water/ionic halide is about 3-40%/10-40%/20-95%/0.1-10% (w/w/w/w) or about 5-10%/10-15%/80-90%/1-10% (w/w/w/w). For example, such solution may include nanoparticles as disclosed herein, about 5% to about 20% by weight sucrose and an ionic halide such as sodium chloride, in a concentration of about 10- 100 mM. In another example, provided herein is a nanoparticle formulation comprising a plurality of disclosed nanoparticles, trehalose, cyclodextrin, and water; wherein the nanoparticles/trehalose/water/cyclodextrin is about 3-40%/1-25%/20-95%/1-25% (w/w/w/w) or about 5-10%/1-25%/80-90%/10-15% (w/w/w/w).

For example, a contemplated solution may include nanoparticles as disclosed herein, about 1% to about 25% by weight of a disaccharide such as trehalose or sucrose (e.g., about 5% to about 25% trehalose or sucrose, e.g. about 10% trehalose or sucrose, or about 15% trehalose or sucrose, e.g. about 5% sucrose) by weight) and a cyclodextrin such as β-cyclodextrin, in a concentration of about 1% to about 25% by weight (*e*.*g*. about 5% to about 20%, e.g. 10% or about 20% by weight, or about 15% to about 20% by weight cyclodextrin). Contemplated formulations may include a plurality of disclosed nanoparticles (*e*.*g*. nanoparticles having PLA-PEG and an active agent), and about 2% to about 15 wt% (or about 4% to about 6wt%, e.g. about 5wt%) sucrose and about 5wt% to about 20% (*e.g.* about 7% wt percent to about 12 wt%, *e.g.* about 10 wt%) of a cyclodextrin, *e.g.*, HPbCD).

The present disclosure relates in part to lyophilized pharmaceutical compositions that, when reconstituted, have a minimal amount of large aggregates. Such large aggregates may have a size greater than about 0.5 µm, greater than about 1 µm, or greater than about 10 µm, and can be undesirable in a reconstituted solution. Aggregate sizes can be measured using a variety of techniques including those indicated in the U.S. Pharmacopeia at 32 <788>, hereby incorporated by reference. The tests outlined in USP 32 <788> include a light obscuration particle count test, microscopic particle count test, laser diffraction, and single particle optical sensing. In one embodiment, the particle size in a given sample is measured using laser diffraction and/or single particle optical sensing.

The USP 32 <788> by light obscuration particle count test sets forth guidelines for sampling particle sizes in a suspension. For solutions with less than or equal to 100 mL, the preparation complies with the test if the average number of particles present does not exceed 6000 per container that are ≥10 µmand 600 per container that are ≥25 µm.

As outlined in USP 32 <788>, the microscopic particle count test sets forth guidelines for determining particle amounts using a binocular microscope adjusted to 100 ± 10x magnification having an ocular micrometer. An ocular micrometer is a circular diameter graticule that consists of a circle divided into quadrants with black reference circles denoting 10 µm and 25 µm when viewed at 100x magnification. A linear scale is provided below the graticule. The number of particles with reference to 10 µm and 25 µm are visually tallied. For solutions with less than or equal to 100 mL, the preparation complies with the test if the average number of particles present does not exceed 3000 per container that are ≥10 µm and 300 per container that are ≥25 µm.

In some embodiments, a 10 mL aqueous sample of a disclosed composition upon reconstitution comprises less than 600 particles per ml having a size greater than or equal to 10 microns; and/or less than 60 particles per ml having a size greater than or equal to 25 microns.

Dynamic light scattering (DLS) may be used to measure particle size, but it relies on Brownian motion so the technique may not detect some larger particles. Laser diffraction relies on differences in the index of refraction between the particle and the suspension media. The technique is capable of detecting particles at the sub-micron to millimeter range. Relatively small (e.g., about 1-5 weight %) amounts of larger particles can be determined in nanoparticle suspensions. Single particle optical sensing (SPOS) uses light obscuration of dilute suspensions to count individual particles of about 0.5 µm. By knowing the particle concentration of the measured sample, the weight percentage of aggregates or the aggregate concentration (particles/mL) can be calculated.

Formation of aggregates can occur during lyophilization due to the dehydration of the surface of the particles. This dehydration can be avoided by using lyoprotectants, such as disaccharides, in the suspension before lyophilization. Suitable disaccharides include sucrose, lactulose, lactose, maltose, trehalose, or cellobiose, and/or mixtures thereof. Other contemplated disaccharides include kojibiose, nigerose, isomaltose, β,β-trehalose, α,β-trehalose, sophorose, laminaribiose, gentiobiose, turanose, maltulose, palatinose, gentiobiulose, mannobiase, melibiose, melibiulose, rutinose, rutinulose, and xylobiose. Reconstitution shows equivalent DLS size distributions when compared to the starting suspension. However, laser diffraction can detect particles of >10 µm in size in some reconstituted solutions. Further, SPOS also may detect >10 µm sized particles at a concentration above that of the FDA guidelines (10⁴-10⁵ particles/mL for >10 µm particles).

In some embodiments, one or more ionic halide salts may be used as an additional lyoprotectant to a sugar, such as sucrose, trehalose or mixtures thereof. Sugars may include disaccharides, monosaccharides, trisaccharides, and/or polysaccharides, and may include other excipients, e.g. glycerol and/or surfactants. Optionally, a cyclodextrin may be included as an additional lyoprotectant. The cyclodextrin may be added in place of the ionic halide salt. Alternatively, the cyclodextrin may be added in addition to the ionic halide salt.

Suitable ionic halide salts may include sodium chloride, calcium chloride, zinc chloride, or mixtures thereof. Additional suitable ionic halide salts include potassium chloride, magnesium chloride, ammonium chloride, sodium bromide, calcium bromide, zinc bromide, potassium bromide, magnesium bromide, ammonium bromide, sodium iodide, calcium iodide, zinc iodide, potassium iodide, magnesium iodide, or ammonium iodide, and/or mixtures thereof. In one embodiment, about 1 to about 15 weight percent sucrose may be used with an ionic halide salt. In one embodiment, the lyophilized pharmaceutical composition may comprise about 10 to about 100 mM sodium chloride. In another embodiment, the lyophilized pharmaceutical composition may comprise about 100 to about 500 mM of divalent ionic chloride salt, such as calcium chloride or zinc chloride. In yet another embodiment, the suspension to be lyophilized may further comprise a cyclodextrin, for example, about 1 to about 25 weight percent of cyclodextrin may be used.

A suitable cyclodextrin may include α-cyclodextrin, β-cyclodextrin, γ-cyclodextrin, or mixtures thereof. Exemplary cyclodextrins contemplated for use in the compositions disclosed herein include hydroxypropyl-β-cyclodextrin (HPbCD), hydroxyethyl-β-cyclodextrin, sulfobutylether-β-cyclodextrin, methyl-β-cyclodextrin, dimethyl-β-cyclodextrin, carboxymethyl-β-cyclodextrin, carboxymethyl ethyl -β-cyclodextrin, diethyl-β-cyclodextrin, triO-alkyl-β-cyclodextrin, glycosyl-β-cyclodextrin, and maltosyl-β-cyclodextrin. In one embodiment, about 1 to about 25 weight percent trehalose (*e.g.* about 10% to about 15%, *e.g.* 5 to about 20% by weight) may be used with cyclodextrin. In one embodiment, the lyophilized pharmaceutical composition may comprise about 1 to about 25 weight percent β-cyclodextrin. An exemplary composition may comprise nanoparticles comprising PLA-PEG, an active/therapeutic agent, about 4% to about 6% (*e.g.* about 5% wt percent) sucrose, and about 8 to about 12 weight percent (*e*.*g*. about 10 wt. %) HPbCD.

In one aspect, a lyophilized pharmaceutical composition is provided comprising disclosed nanoparticles, wherein upon reconstitution of the lyophilized pharmaceutical composition at a nanoparticle concentration of about 50 mg/mL, in less than or about 100 mL of an aqueous medium, the reconstituted composition suitable for parenteral administration comprises less than 6000, such as less than 3000, microparticles of greater than or equal to 10 microns; and/or less than 600, such as less than 300, microparticles of greater than or equal to 25 microns.

The number of microparticles can be determined by means such as the USP 32 <788> by light obscuration particle count test, the USP 32 <788> by microscopic particle count test, laser diffraction, and single particle optical sensing.

In an aspect, a pharmaceutical composition suitable for parenteral use upon reconstitution is provided comprising a plurality of therapeutic particles each comprising a copolymer having a hydrophobic polymer segment and a hydrophilic polymer segment; an active agent; a sugar; and a cyclodextrin.

For example, the copolymer may be poly(lactic) acid-*block*-poly(ethylene)glycol copolymer. Upon reconstitution, a 100 mL aqueous sample may comprise less than 6000 particles having a size greater than or equal to 10 microns; and less than 600 particles having a size greater than or equal to 25 microns.

The step of adding a disaccharide and an ionic halide salt may comprise adding about 5 to about 15 weight percent sucrose or about 5 to about 20 weight percent trehalose (e.g., about 10 to about 20 weight percent trehalose), and about 10 to about 500 mM ionic halide salt. The ionic halide salt may be selected from sodium chloride, calcium chloride, and zinc chloride, or mixtures thereof. In an embodiment, about 1 to about 25 weight percent cyclodextrin is also added.

In another embodiment, the step of adding a disaccharide and a cyclodextrin may comprise adding about 5 to about 15 weight percent sucrose or about 5 to about 20 weight percent trehalose (*e*.*g*., about 10 to about 20 weight percent trehalose), and about 1 to about 25 weight percent cyclodextrin. In an embodiment, about 10 to about 15 weight percent cyclodextrin is added. The cyclodextrin may be selected from α-cyclodextrin, β-cyclodextrin, γ-cyclodextrin, or mixtures thereof.

In another aspect, a method of preventing substantial aggregation of particles in a pharmaceutical nanoparticle composition is provided comprising adding a sugar and a salt to the lyophilized formulation to prevent aggregation of the nanoparticles upon reconstitution. In an embodiment, a cyclodextrin is also added to the lyophilized formulation. In yet another aspect, a method of preventing substantial aggregation of particles in a pharmaceutical nanoparticle composition is provided comprising adding a sugar and a cyclodextrin to the lyophilized formulation to prevent aggregation of the nanoparticles upon reconstitution.

A contemplated lyophilized composition may have a therapeutic particle concentration of greater than about 40 mg/mL. The formulation suitable for parenteral administration may have less than about 600 particles having a size greater than 10 microns in a 10 mL dose. Lyophilizing may comprise freezing the composition at a temperature of greater than about -40 °C, or *e.g.* less than about -30 °C, forming a frozen composition; and drying the frozen composition to form the lyophilized composition. The step of drying may occur at about 50 mTorr at a temperature of about -25 to about -34 °C, or about -30 to about -34 °C.

### Methods of Treatment

In some embodiments, contemplated nanoparticles may be used to treat, alleviate, ameliorate, relieve, delay onset of, inhibit progression of, reduce severity of, and/or reduce incidence of one or more symptoms or features of a disease, disorder, and/or condition. In some embodiments, contemplated nanoparticles may be used to treat solid tumors, *e*.*g*., cancer and/or cancer cells.

The term "cancer" includes pre-malignant as well as malignant cancers. Cancers include, but are not limited to, blood (*e*.*g*., leukemia, chronic myelogenous leukemia, chronic myelomonocytic leukemia, acute lymphoblastic leukemia, Philadelphia chromosome positive acute lymphoblastic leukemia, mantle cell lymphoma, non-Hodgkin's lymphoma, Hodgkin's lymphoma), prostate, gastric cancer, colorectal cancer, skin cancer, *e*.*g*., melanomas or basal cell carcinomas, lung cancer (*e*.*g*., non-small cell lung cancer), breast cancer, cancers of the head and neck, bronchus cancer, pancreatic cancer, urinary bladder cancer, brain or central nervous system cancer, peripheral nervous system cancer, esophageal cancer, cancer of the oral cavity or pharynx, liver cancer (*e.g*., hepatocellular carcinoma), kidney cancer (*e.g*., renal cell carcinoma, acute nephroblastoma), testicular cancer, biliary tract cancer, small bowel or appendix cancer, gastrointestinal stromal tumor, salivary gland cancer, thyroid gland cancer, adrenal gland cancer, osteosarcoma, chondrosarcoma, cancer of hematological tissues, and the like. "Cancer cells" can be in the form of a tumor (*i.e*., a solid tumor), exist alone within a subject (*e.g*., leukemia cells), or be cell lines derived from a cancer.

Cancer can be associated with a variety of physical symptoms. Symptoms of cancer generally depend on the type and location of the tumor. For example, lung cancer can cause coughing, shortness of breath, and chest pain, while colon cancer often causes diarrhea, constipation, and blood in the stool. However, to give but a few examples, the following symptoms are often generally associated with many cancers: fever, chills, night sweats, cough, dyspnea, weight loss, loss of appetite, anorexia, nausea, vomiting, diarrhea, anemia, jaundice, hepatomegaly, hemoptysis, fatigue, malaise, cognitive dysfunction, depression, hormonal disturbances, neutropenia, pain, non-healing sores, enlarged lymph nodes, peripheral neuropathy, and sexual dysfunction.

In one aspect, a method for the treatment of cancer is provided. In some embodiments, the treatment of cancer comprises administering a therapeutically effective amount of inventive particles to a subject in need thereof, in such amounts and for such time as is necessary to achieve the desired result. In certain embodiments, a "therapeutically effective amount" of an inventive particle is that amount effective for treating, alleviating, ameliorating, relieving, delaying onset of, inhibiting progression of, reducing severity of, and/or reducing incidence of one or more symptoms or features of cancer.

In one aspect, a method for administering inventive compositions to a subject suffering from cancer is provided. In some embodiments, nanoparticles may be administered to a subject in such amounts and for such time as is necessary to achieve the desired result (*i.e*., treatment of cancer). In certain embodiments, a "therapeutically effective amount" of a contemplated nanoparticle is that amount effective for treating, alleviating, ameliorating, relieving, delaying onset of, inhibiting progression of, reducing severity of, and/or reducing incidence of one or more symptoms or features of cancer.

In some embodiments, the contemplated nanoparticles may be administered to a patient in need thereof as part of a combination therapy. For example, in certain embodiments, the contemplated nanoparticles may be administered as part of a chemotherapy regimen. Non-limiting examples of chemotherapy regimens include CHOP (i.e., Cyclophosphamide, Hydroxydaunorubicin, Oncovin (*i.e*., vincristine), and Prednisone), MOPP (i.e., Mustargen, Oncovin, Procarbazine, and Prednisone), COPP (i.e., Cyclophosphamide, Oncovin, Procarbazine, and Prednisone), BEACOPP (i.e., Bleomycin, Etoposide, Adriamycin, Cyclophosphamide, Oncovin, Procarbazine, and Prednisone), and Stanford V (i.e., a mustard derivative such as Cyclophosphamide, Mechlorethamine or Ifosfamide; Doxorubicin; Vinblastine; Vincristine; Bleomycin; Etoposide; and Prednisone), where the contemplated vincristine nanoparticles may be used in place of or used in addition to the vincristine component of said chemotherapy regimens. In some embodiments, the contemplated vincristine nanoparticles may be administered in combination with dexamethasone and/or L-asparaginase or in combination with prednisone.

Inventive therapeutic protocols involve administering a therapeutically effective amount of a contemplated nanoparticle to a healthy individual (*i.e*., a subject who does not display any symptoms of cancer and/or who has not been diagnosed with cancer). For example, healthy individuals may be "immunized" with a contemplated nanoparticle prior to development of cancer and/or onset of symptoms of cancer; at risk individuals (*e*.*g*., patients who have a family history of cancer; patients carrying one or more genetic mutations associated with development of cancer; patients having a genetic polymorphism associated with development of cancer; patients infected by a virus associated with development of cancer; patients with habits and/or lifestyles associated with development of cancer; *etc*.) can be treated substantially contemporaneously with (*e*.*g*., within 48 hours, within 24 hours, or within 12 hours of) the onset of symptoms of cancer. Of course, individuals known to have cancer may receive inventive treatment at any time.

In other embodiments, disclosed nanoparticles can be used to inhibit the growth of cancer cells, *e.g.*, lung cancer cells. As used herein, the term "inhibits growth of cancer cells" or "inhibiting growth of cancer cells" refers to any slowing of the rate of cancer cell proliferation and/or migration, arrest of cancer cell proliferation and/or migration, or killing of cancer cells, such that the rate of cancer cell growth is reduced in comparison with the observed or predicted rate of growth of an untreated control cancer cell. The term "inhibits growth" can also refer to a reduction in size or disappearance of a cancer cell or tumor, as well as to a reduction in its metastatic potential. Preferably, such an inhibition at the cellular level may reduce the size, deter the growth, reduce the aggressiveness, or prevent or inhibit metastasis of a cancer in a patient. Those skilled in the art can readily determine, by any of a variety of suitable indicia, whether cancer cell growth is inhibited.

Inhibition of cancer cell growth may be evidenced, for example, by arrest of cancer cells in a particular phase of the cell cycle, *e*.*g*., arrest at the G2/M phase of the cell cycle. Inhibition of cancer cell growth can also be evidenced by direct or indirect measurement of cancer cell or tumor size. In human cancer patients, such measurements generally are made using well known imaging methods such as magnetic resonance imaging, computerized axial tomography and X-rays. Cancer cell growth can also be determined indirectly, such as by determining the levels of circulating carcinoembryonic antigen, prostate specific antigen or other cancer-specific antigens that are correlated with cancer cell growth. Inhibition of cancer growth is also generally correlated with prolonged survival and/or increased health and well-being of the subj ect.

Also provided herein are methods of administering to a patient a nanoparticle disclosed herein including an active agent, wherein, upon administration to a patient, such nanoparticles substantially reduces the volume of distribution and/or substantially reduces free Cₘₐₓ, as compared to administration of the agent alone (*i.e*., not as a disclosed nanoparticle).

### EXAMPLES

The invention now being generally described, it will be more readily understood by reference to the following examples which are included merely for purposes of illustration of certain aspects and embodiments, and are not intended to limit the invention in any way.

### EXAMPLE 1: General Preparation of Vincristine-Containing Nanoparticles

An organic phase is formed composed of a mixture of drug (e.g., vincristine), polymer (e.g., poly(lactic acid)-poly(ethylene glycol) diblock copolymer (PLA-PEG)), a solvent, and, optionally, a hydrophobic acid. The organic phase is mixed with an aqueous phase at approximately a 1:5 ratio (oil phase:aqueous phase) where the aqueous phase is composed of a surfactant, some dissolved solvent, and, optionally, a hydrophobic acid. Approximately 20% solids in the organic phase is used.

The primary, coarse emulsion is formed by the combination of the two phases using a homogenizer. The course emulsion is fed through a high pressure homogenizer (110S) with pressure set at 46 psi on gauge for 2 discrete passes to form a nanoemulsion (fine emulsion).

The nanoemulsion is poured into a quench (D.I. water optionally containing a water-soluble hydrophobic acid) at less than 5 °C while stirring on stir plate to form a quenched phase. The ratio of quench to emulsion is 10:1. To the quenched phase is added Tween 80 in water (35% (w/w)) at a ratio of 100:1 Tween 80 to drug.

The quenched phase is concentrated using tangential flow filtration (TFF) with a 300 kDa Pall cassette (2 membrane) to form a nanoparticle concentrate of ∼200 mL. The nanoparticle concentrate is diafiltered with -20 diavolumes (4 L) of cold DI water. The volume of the diafiltered nanoparticle concentrate is reduced to a minimal volume. Cold water (100 mL) is added to the vessel and pumped through the membrane to rinse and form a slurry. The final slurry (∼100 mL) is collected in a glass vial.

Determination of solids concentration of unfiltered final slurry. To a tared 20 mL scintillation vial is added a volume of final slurry, which is dried under vacuum on a lyophilizer/oven. The weight of nanoparticles in the volume of dried slurry is determined. To the final slurry is added concentrated sucrose (0.666 g/g) to attain 10% sucrose.

Determination of solids concentration of 0.45 µm filtered final slurry. A portion of the final slurry sample is filtered through a 0.45µm syringe filter before addition of sucrose. To a tared 20 mL scintillation vial is added a volume of filtered sample, which is dried under vacuum using a lyophilizer/oven. The remaining sample of unfiltered final slurry with sucrose is frozen.

### EXAMPLE 2: Therapeutic Nanoparticles Containing Vincristine

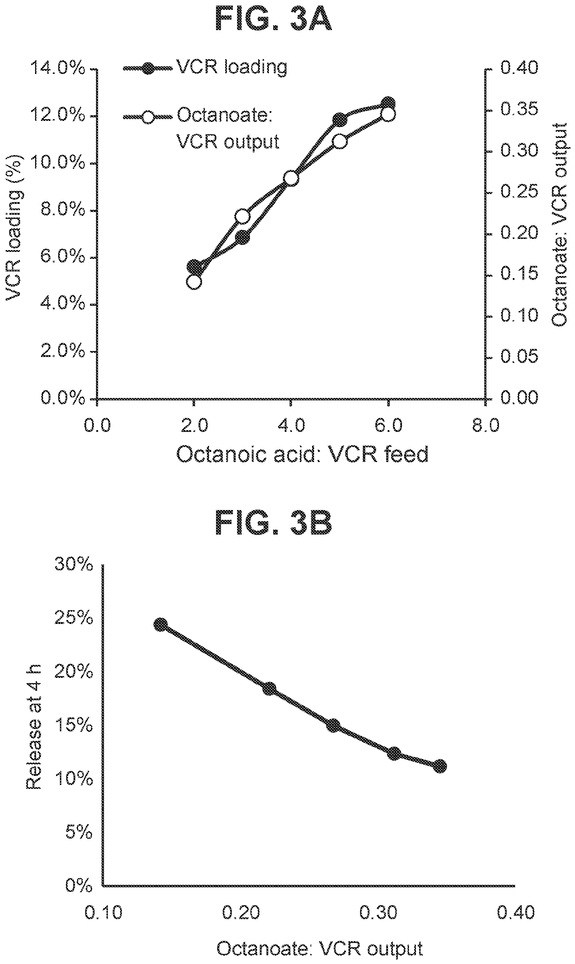

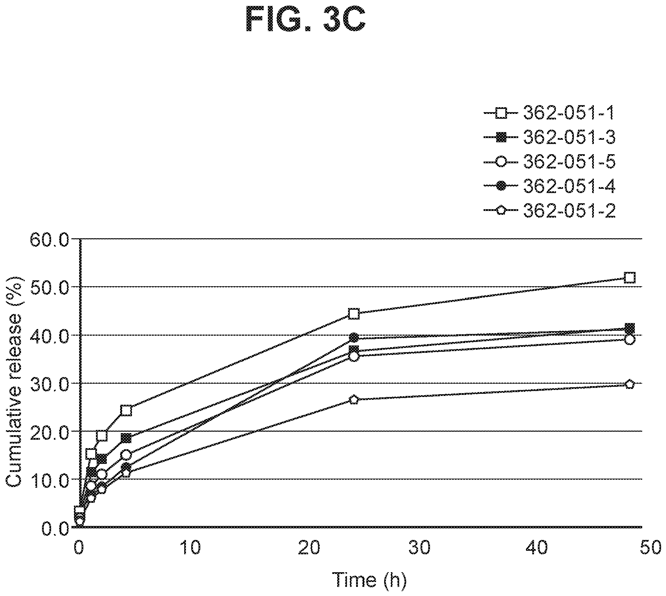

The nanoparticles described below were prepared using a process as described in Example 1, using a water-soluble hydrophobic acid as part of the organic phase of the emulsion process. This procedure can improve drug encapsulation efficiency and drug loading and slow the release of drug as shown in Tables 1-6 and FIGs. 3A, 3B, and 3C.

**Table 1. Characteristics of nanoparticles prepared with decanoic acid in the organic phase.**

| Batch# | Drug theoretical loading | Solids | Ethyl acetate (EA) | Benzyl alcohol (BA) (7.5% in water) | Type of aqueous phase | Surfactant | Pressure (psi) | Feeding molar ratio acid/drug |
|---|---|---|---|---|---|---|---|---|
| 362-017-1 | 19% | 20% | 65% | 35% | 2% BA, 4% EA | Brij S 100 (0.05%) | 9320 | 1.0 |
| 362-017-2 | 18% | 20% | 65% | 35% | 2% BA, 4% EA | Brij S 100 (0.05%) | 9320 | 2.0 |
| 362-017-3 | 17% | 20% | 65% | 35% | 2% BA, 4% EA | Brij S 100 (0.05%) | 9320 | 3.0 |
| 362-017-4 | 17% | 20% | 65% | 35% | 2% BA, 4% EA | Brij S 100 (0.05%) | 9320 | 4.0 |

**Table 2. Characteristics of nanoparticles prepared with decanoic acid in the organic phase.**

| Batch # | Drug loading | Size (nm) | Encapsulation efficiency | Actual molar ratio acid:drug in NP |
|---|---|---|---|---|
| 362-017-1 | 10.3 | 93 | 55% | 0.55 |
| 362-017-2 | 14.7 | 97 | 81% | 0.92 |
| 362-017-3 | 17.1 | 97 | 98% | 1.02 |
| 362-017-4 | 17.1 | 97 | 102% | 1.33 |

**Table 3. Characteristics of nanoparticles prepared with decanoic acid, octanoic acid, hexanoic acid, or isovaleric acid in the organic phase.**

| Batch # | Drug theoretical loading | Solids | Ethyl acetate (EA) | Benzyl alcohol (BA) (7.5% in water) | Type of aqueous phase | Surfactant | Pressure (psi) | Acid (counterion) | Feeding molar ratio acid/drug |
|---|---|---|---|---|---|---|---|---|---|
| 362-043-1 | 17% | 20% | 65% | 35% | 2% BA, 4% EA | Brij S 100 (0.05%) | 9320 | Decanoic acid | 3.0 |
| 362-043-2 | 17% | 20% | 65% | 35% | 2% BA, 4% EA | Brij S 100 (0.05%) | 9320 | Decanoic acid | 3.0 |
| 362-043-3 | 18% | 20% | 65% | 35% | 2% BA, 4% EA | Brij S 100 (0.05%) | 9320 | Octanoic acid | 3.0 |
| 362-043-4 | 18% | 20% | 65% | 35% | 2% BA, 4% EA | Brij S 100 (0.05%) | 9320 | Octanoic acid | 3.0 |
| 362-043-5 | 18% | 20% | 65% | 35% | 2% BA, 4% EA | Brij S 100 (0.05%) | 9320 | Hexanoic acid | 3.0 |
| 362-043-6 | 18% | 20% | 65% | 35% | 2% BA, 4% EA | Brij S 100 (0.05%) | 9320 | Hexanoic acid | 3.0 |
| 362-043-7 | 18% | 20% | 65% | 35% | 2% BA, 4% EA | Brij S 100 (0.05%) | 9320 | Isovaleri c acid | 3.0 |

**Table 4. Characteristics of nanoparticles prepared with decanoic acid, octanoic acid, hexanoic acid, or isovaleric acid in the organic phase.**

| Batch # | Drug loading | Size (nm) | Encapsulation efficiency | Actual molar ratio acid:drug in NP |
|---|---|---|---|---|
| 362-043-1 | 17.7% | 93 | 102% | 0.81 |
| 362-043-2 | 18.0% | 94 | 103% | 0.78 |
| 362-043-3 | 14.1% | 92 | 80% | 0.17 |
| 362-043-4 | 9.6% | 95 | 54% | 0.14 |
| 362-043-5 | 3.0% | 95 | 16% | 2.90 |
| 362-043-6 | 3.2% | 100 | 18% | 2.89 |
| 362-043-7 | 2.6% | 97 | 14% | --- |

**Table 5. Characteristics of nanoparticles prepared with octanoic acid in the organic phase.**

| Batc h# | Drug theoretical loading | Solids | Ethyl acetate (EA) | Benzyl alcohol (BA) (7.5% in water) | Type of aqueous phase | Surfactant | Pressure (psi) | Feeding molar ratio acid/drug |
|---|---|---|---|---|---|---|---|---|
| 362- 051- 1 | 18% | 22% | 65% | 35% | 2% BA, 4% EA | Brij S 100 (0.05%) | 9320 | 2.0 |
| 362-051-3 | 18% | 22% | 65% | 35% | 2% BA, 4% EA | Brij S 100 (0.05%) | 9320 | 3.0 |
| 362-051-5 | 17% | 23% | 65% | 35% | 2% BA, 4% EA | Brij S 100 (0.05%) | 9320 | 4.0 |
| 362-051-4 | 17% | 23% | 65% | 35% | 2% BA, 4% EA | Brij S 100 (0.05%) | 9320 | 5.0 |
| 362-051-2 | 16% | 24% | 65% | 35% | 2% BA, 4% EA | Brij S 100 (0.05%) | 9320 | 6.0 |

**Table 6. Characteristics of nanoparticles prepared with octanoic acid in the organic phase.**

| Batch # | Drug loading | Size (nm) | Encapsulation efficiency | Actual molar ratio acid:drug in NP |
|---|---|---|---|---|
| 362-051-1 | 5.6% | 95 | 31% | 0.14 |
| 362-051-3 | 6.9% | 94 | 39% | 0.22 |
| 362-051-5 | 9.4% | 95 | 54% | 0.27 |
| 362-051-4 | 11.8% | 98 | 71% | 0.31 |
| 362-051-2 | 12.5% | 100 | 77% | 0.35 |

FIG. 3A shows vincristine (VCR) drug loading and output ratio of octanoate to vincristine (*i.e*., ion pairing of octanoate with VCR) versus the ratio of octanoate to vincristine used in the organic phase. As can be seen in the figure, increasing the amount of octanoic acid in the organic phase resulted in increasing VCR drug loading and increasing ion pairing of VCR with octanoate.

FIG. 3B shows the amount of VCR released at 4 hours versus output ratio of octanoate to vincristine. As can be seen in the figure, increasing the amount of octanoic acid in the organic phase resulted in slower release of VCR from the nanoparticles.

FIG. 3C shows the cumulative amount of VCR released over time for the formulations described in Tables 5 and 6. As can be seen in the figure, increasing the amount of octanoic acid in the organic phase resulted in slower release of VCR from the nanoparticles.

### EXAMPLE 3: Therapeutic Nanoparticles Containing Vincristine

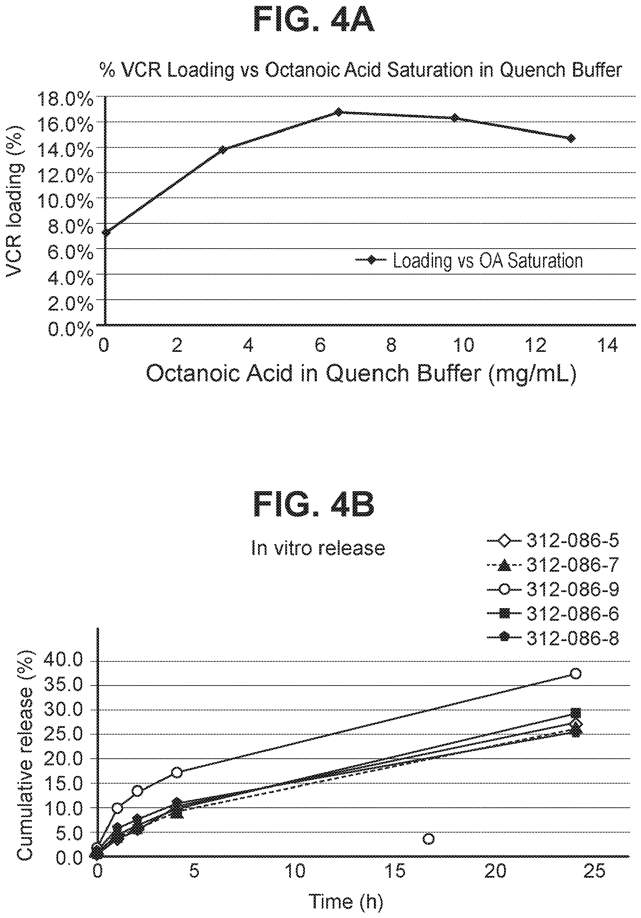

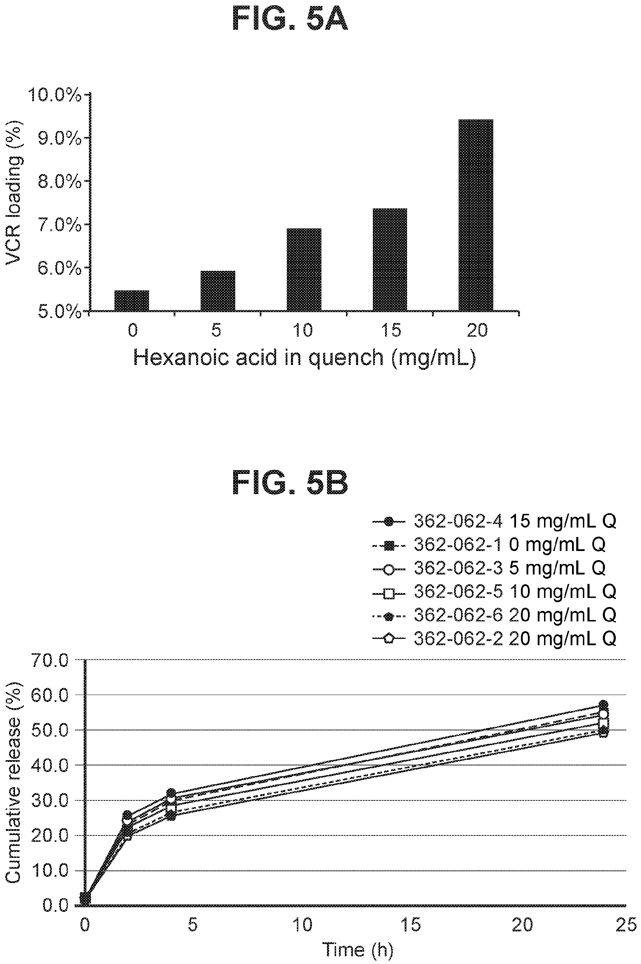

The nanoparticles described below were prepared using a process as described in Example 1, using a water-soluble hydrophobic acid as part of the organic phase and quench of the emulsion process. This procedure can improve drug encapsulation efficiency and drug loading and slow the release of drug as shown in Tables 7-10 and FIGs. 4A, 4B, 5A, and 5B.

**Table 7. Characteristics of nanoparticles prepared with octanoic acid in the organic phase or both the organic phase and the quench.**

| Batch # | Drug theoretical loading | Solids | Ethyl acetate (EA) | Benzyl alcohol (BA) (7.5% in water) | Type of aqueous phase | Surfactant | Pressure (psi) | Feeding molar ratio acid/drug | Octanoic acid concentration in quench buffer (mg/mL) |
|---|---|---|---|---|---|---|---|---|---|
| 312-086-5 | 17% | 20% | 65% | 35% | 2% BA, 4% EA | Brij S 100 (0.1%) | 9320 | 3.0 | 13 |
| 312-086-6 | 17% | 20% | 65% | 35% | 2% BA, 4% EA | Brij S 100 (0.1%) | 9320 | 3.0 | 9.75 |
| 312-086-7 | 17% | 20% | 65% | 35% | 2% BA, 4% EA | Brij S 100 (0.1%) | 9320 | 3.0 | 6.5 |
| 312-086-8 | 17% | 20% | 65% | 35% | 2% BA, 4% EA | Brij S 100 (0.1%) | 9320 | 3.0 | 3.25 |
| 312-086-9 | 17% | 20% | 65% | 35% | 2% BA, 4% EA | Brij S 100 (0.1%) | 9320 | 3.0 | 0 |

**Table 8. Characteristics of nanoparticles prepared with octanoic acid in the organic phase or both the organic phase and the quench.**

| Batch # | Drug loading | Size (nm) | Encapsulation efficiency | Actual molar ratio acid:drug in NP |
|---|---|---|---|---|
| 312-086-5 | 14.7% | 101.1 | 85% | 0.96 |
| 312-086-6 | 16.3% | 94.5 | 94% | 1.04 |
| 312-086-7 | 16.8% | 97.4 | 96% | 0.96 |
| 312-086-8 | 13.8% | 94.1 | 80% | 0.71 |
| 312-086-9 | 7.3% | 93.2 | 42% | 0.20 |

FIG. 4A shows vincristine (VCR) drug loading versus octanoic acid concentration in the quench. As can be seen in the figure, drug loading and encapsulation efficiency reached a maximum at about 7 mg/mL octanoic acid in the quench.

FIG. 4B shows the cumulative amount of VCR released over time for the formulations described in Tables 7 and 8. As can be seen in the figure, including octanoic acid in the quench resulted in slower release of VCR from the nanoparticles as compared to nanoparticles prepared without octanoic acid in the quench.

**Table 9. Characteristics of nanoparticles prepared with hexanoic acid in the organic phase or both the organic phase and the quench.**

| Batch # | Drug theoretical loading | Solids | Ethyl acetate (EA) | Benzyl alcohol (BA) (7.5% in water) | Type of aqueous phase | Surfactant | Pressure (psi) | Feeding molar ratio acid/drug | Hexanoic acid concentration in quench buffer (mg/mL) |
|---|---|---|---|---|---|---|---|---|---|
| 362-062-1 | 18% | 22% | 65% | 35% | 2% BA, 4% EA | Brij S 100 (0.05%) | 9320 | 4.0 | 0 |
| 362-062-3 | 18% | 22% | 65% | 35% | 2% BA, 4% EA | Brij S 100 (0.05%) | 9320 | 4.0 | 5 |
| 362-062-5 | 18% | 22% | 65% | 35% | 2% BA, 4% EA | Brij S 100 (0.05%) | 9320 | 4.0 | 10 |
| 362-062-4 | 18% | 22% | 65% | 35% | 2% BA, 4% EA | Brij S 100 (0.05%) | 9320 | 4.0 | 15 |
| 362-062-2 | 18% | 22% | 65% | 35% | 2% BA, 4% EA | Brij S 100 (0.05%) | 9320 | 4.0 | 20 |
| 362-062-6 | 20% | 20% | 65% | 35% | 2% BA, 4% EA | Brij S 100 (0.05%) | 9320 | 0.0 | 20 |

**Table 10. Characteristics of nanoparticles prepared with hexanoic acid in the organic phase or both the organic phase and the quench.**

| Batch # | Drug loading | Size (nm) | Encapsulation efficiency | Actual molar ratio acid:drug in NP |
|---|---|---|---|---|
| 362-062-1 | 5.5% | 96 | 31% | 0.08 |
| 362-062-3 | 5.9% | 98 | 34% | 0.33 |
| 362-062-5 | 6.9% | 98 | 39% | 0.39 |
| 362-062-4 | 7.4% | 102 | 42% | 0.56 |
| 362-062-2 | 9.4% | 98 | 54% | 0.50 |
| 362-062-6 | 8.5% | 95 | 44% | 0.53 |

FIG. 5A shows vincristine (VCR) drug loading versus hexanoic acid concentration in the quench. As can be seen in the figure, drug loading increased with increasing hexanoic acid in the quench.

FIG. 5B shows the cumulative amount of VCR released over time for the formulations described in Tables 9 and 10. As can be seen in the figure, nanoparticles prepared with hexanoic acid in both the organic phase and the quench had a similar release profile as compared to nanoparticles prepared with hexanoic acid only in the quench.

### EXAMPLE 4: Therapeutic Nanoparticles Containing Vincristine

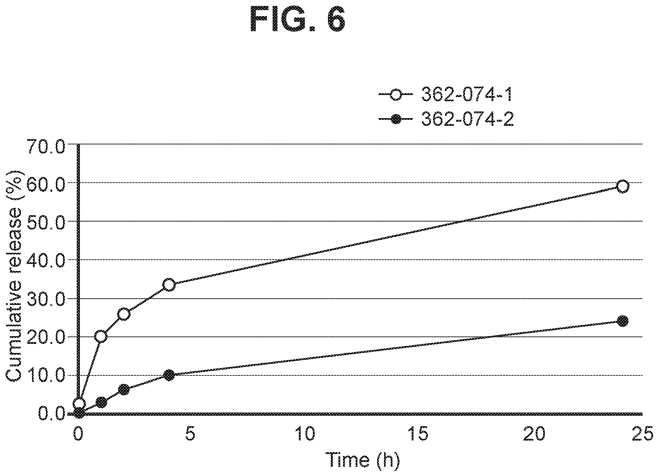

The nanoparticles described below were prepared using a process as described in Example 1, using a water-soluble hydrophobic acid as part of the quench of the emulsion process. This procedure can improve drug encapsulation efficiency and drug loading and slow the release of drug as shown in Tables 11-12 and FIG. 6.

**Table 11. Characteristics of nanoparticles prepared with hexanoic acid in the quench or no hexanoic acid in the quench.**

| Batch # | Drug theoretical loading | Solids | Ethyl acetate (EA) | Benzyl alcohol (BA) (7.5% in water) | Type of aqueous phase | Surfactant | Pressure (psi) | Feeding molar ratio acid/drug | Hexanoic acid concentration in quench buffer (mg/mL) |
|---|---|---|---|---|---|---|---|---|---|
| 362-074-1 | 20% | 20% | 65% | 35% | 2% BA, 4% EA | Brij S 100 (0.05%) | 9320 | 0.0 | 0 |
| 362-074-2 | 20% | 20% | 65% | 35% | 2% BA, 4% EA | Brij S 100 (0.05%) | 9320 | 0.0 | 50 |
| 362-074-3 | 20% | 20% | 65% | 35% | 2% BA, 4% EA | Brij S 100 (0.05%) | 9320 | 0.0 | 100 |
| 362-074-4 | 20% | 20% | 65% | 35% | 2% BA, 4% EA | Brij S 100 (0.05%) | 9320 | 0.0 | 150 |
| 362-074-5 | 20% | 20% | 65% | 35% | 2% BA, 4% EA | Brij S 100 (0.05%) | 9320 | 0.0 | 200 |

**Table 12. Characteristics of nanoparticles prepared with hexanoic acid in the quench or no hexanoic acid in the quench.**

| Batch # | Drug loading | Size (nm) | Encapsulation efficiency | Actual molar ratio acid:drug in NP |
|---|---|---|---|---|
| 362-074-1 | 2.1% | 101 | 11% | 0.00 |
| 362-074-2 | 12.9% | 95 | 66% | 0.59 |
| 362-074-3 | 0.1% | 74 | 1% | 28.7 |
| 362-074-4 | 0.1% | 52 | 1% | 26.0 |
| 362-074-5 | 0.1% | 69 | 0% | 35.1 |

FIG. 6 shows the cumulative amount of VCR released over time for two formulations described in Tables 11 and 12. As can be seen in the figure, nanoparticles prepared with hexanoic acid in the quench and no hexanoic acid in the organic phase had a much slower release profile as compared to nanoparticles prepared without hexanoic acid in either the quench or the organic phase.

## Claims

1. A method of preparing a plurality of therapeutic nanoparticles, comprising:
combining a first polymer and a therapeutic agent with an organic solvent, and optionally a first substantially hydrophobic acid, to form a first organic phase having about 1 to about 50% solids;
combining the first organic phase with a first aqueous phase to form an emulsion phase;
combining the emulsion phase with a quench to form a quenched phase comprising the plurality of therapeutic nanoparticles, wherein the quench comprises water and a second substantially hydrophobic acid having at least some water solubility; and
recovering the therapeutic nanoparticles by filtration.

2. The method of claim 1, wherein the first or second substantially hydrophobic acid are independently selected from the group consisting of caproic acid, enanthic acid, caprylic acid, pelargonic acid, capric acid, undecanoic acid, lauric acid, tridecanoic acid, myristic acid, pentadecanoic acid, palmitic acid, cinnamic acid, phenylacetic acid, dodecylbenzenesulfonic acid, dioctyl sulfosuccinic acid, dioleoyl phosphatidic acid, and chenodeoxycholic acid, ursodeoxycholic acid, deoxycholic acid, cholic acid, and lithocholic acid, and combinations thereof.

3. The method of claim 1 or 2, wherein the molar ratio of the substantially hydrophobic acid to the therapeutic agent in the nanoparticle is about 0.1:1 to about 1.2:1.

4. The method of any one of claim 1 or 2, wherein the molar ratio of the substantially hydrophobic acid to the therapeutic agent in the nanoparticle is about 0.75:1 to about 1.2:1.

5. The method of any one of claims 1-4, wherein the first organic phase comprises a substantially hydrophobic acid having at least some water solubility.

6. The method of any one of claims 1-4, wherein the first aqueous phase comprises a substantially hydrophobic acid having at least some water solubility.

7. The method of any one of claims 1-6, wherein the emulsion phase comprises a substantially hydrophobic acid having at least some water solubility and wherein the emulsion phase includes a molar ratio of the substantially hydrophobic acid to the therapeutic agent of about 0.5:1 to about 6:1.

8. The method of any one of claims 1-7, wherein the method has an encapsulation efficiency of between about 75% and about 100%.

9. The method of any one of claims 1-8, wherein the therapeutic nanoparticles comprise about 5 to about 20 weight percent of the therapeutic agent.

10. The method of any one of claims 1-8, wherein the therapeutic nanoparticles comprise about 10 to about 30 weight percent of the therapeutic agent.

11. The method of any one of claims 1-10, wherein the quench has a temperature of about 0 °C to about 5 °C.

12. The method of any one of claims 1-11, wherein the quench:emulsion ratio is about 2:1 to about 40:1.

13. The method of any one of claims 1-12, further comprising adding a drug solubilizer to the quenched phase to form a solubilized phase of unencapsulated therapeutic agent.

14. The method of any one of claims 1-13, wherein the therapeutic nanoparticles substantially immediately release less than about 5% of the therapeutic agent when placed in a phosphate buffer solution at 25 °C.

15. A plurality of therapeutic nanoparticles prepared by a process comprising:
combining a first polymer and a therapeutic agent with an organic solvent to form a first organic phase having about 1 to about 50% solids;
combining the first organic phase with a first aqueous phase to form an emulsion phase;
combining the emulsion phase with a quench to form a quenched phase comprising the plurality of therapeutic nanoparticles, wherein the quench comprises water and a substantially hydrophobic acid having at least some water solubility; and
recovering the therapeutic nanoparticles by filtration.

## Patentansprüche

1. Verfahren zur Herstellung einer Vielzahl therapeutischer Nanopartikel, umfassend:
Kombinieren eines ersten Polymers und eines therapeutischen Mittels mit einem organischen Lösungsmittel und gegebenenfalls einer ersten im Wesentlichen hydrophoben Säure unter Bildung einer ersten organischen Phase, die etwa 1 bis etwa 50% Feststoffe hat;
Kombinieren der ersten organischen Phase mit einer ersten wässrigen Phase unter Bildung einer Emulsionsphase;
Kombinieren der Emulsionsphase mit einem Quenchmittel, um eine gequenchte Phase zu bilden, die die Vielzahl therapeutischer Nanopartikel umfasst, wobei das Quenchmittel Wasser und eine zweite im Wesentlichen hydrophobe Säure, die wenigstens etwas Wasserlöslichkeit hat, umfasst; und
Gewinnen der therapeutischen Nanopartikel durch Filtration.

2. Verfahren nach Anspruch 1, wobei die erste oder zweite im Wesentlichen hydrophobe Säure unabhängig ausgewählt wird aus der Gruppe, bestehend aus Capronsäure, Önanthsäure, Caprylsäure, Nonansäure, Caprinsäure, Undecansäure, Laurinsäure, Tridecansäure, Myristinsäure, Pentadecansäure, Palmitinsäure, Zimtsäure, Phenylessigsäure, Dodecylbenzolsulfonsäure, Dioctylsulfobernsteinsäure, Dioleoylphosphatidsäure und Chenodesoxycholsäure, Ursodesoxycholsäure, Desoxycholsäure, Cholsäure und Lithocholsäure und Kombinationen davon.

3. Verfahren nach Anspruch 1 oder 2, wobei das Molverhältnis der im Wesentlichen hydrophoben Säure zu dem therapeutischen Mittel in dem Nanopartikel etwa 0,1:1 bis etwa 1,2:1 ist.

4. Verfahren nach einem der Ansprüche 1 oder 2, wobei das Molverhältnis der im Wesentlichen hydrophoben Säure zu dem therapeutischen Mittel in dem Nanopartikel etwa 0,75:1 bis etwa 1,2:1 ist.

5. Verfahren nach einem der Ansprüche 1-4, wobei die erste organische Phase eine im Wesentlichen hydrophobe Säure, die wenigstens etwas Wasserlöslichkeit hat, umfasst.

6. Verfahren nach einem der Ansprüche 1-4, wobei die erste wässrige Phase eine im Wesentlichen hydrophobe Säure, die wenigstens etwas Wasserlöslichkeit hat, umfasst.

7. Verfahren nach einem der Ansprüche 1-6, wobei die Emulsionsphase eine im Wesentlichen hydrophobe Säure, die wenigstens etwas Wasserlöslichkeit hat, umfasst, und wobei die Emulsionsphase ein Molverhältnis der im Wesentlichen hydrophoben Säure zu dem therapeutischen Mittel von etwa 0,5:1 bis etwa 6:1 umfasst.

8. Verfahren nach einem der Ansprüche 1-7, wobei das Verfahren eine Einkapselungseffizienz von zwischen etwa 75% und etwa 100% hat.

9. Verfahren nach einem der Ansprüche 1-8, wobei die therapeutischen Nanopartikel etwa 5 bis etwa 20 Gewichtsprozent des therapeutischen Mittels umfassen.

10. Verfahren nach einem der Ansprüche 1-8, wobei die therapeutischen Nanopartikel etwa 10 bis etwa 30 Gewichtsprozent des therapeutischen Mittels umfassen.

11. Verfahren nach einem der Ansprüche 1-10, wobei das Quenchmittel eine Temperatur von etwa 0°C bis etwa 5°C hat.

12. Verfahren nach einem der Ansprüche 1-11, wobei das Quenchmittel:Emulsion-Verhältnis etwa 2:1 bis etwa 40:1 ist.

13. Verfahren nach einem der Ansprüche 1-12, außerdem umfassend Zugeben eines Wirkstoffsolubilisators zu der gequenchten Phase, um eine solubilisierte Phase von nicht eingekapseltem therapeutischen Mittel zu bilden.

14. Verfahren nach einem der Ansprüche 1-13, wobei die therapeutischen Nanopartikel weniger als etwa 5% des therapeutischen Mittels im Wesentlichen unverzüglich freisetzen, wenn sie in eine Phosphatpufferlösung mit 25°C platziert werden.

15. Vielzahl therapeutischer Nanopartikel, die durch ein Verfahren hergestellt wurden, das umfasst:
Kombinieren eines ersten Polymers und eines therapeutischen Mittels mit einem organischen Lösungsmittel unter Bildung einer ersten organischen Phase, die etwa 1 bis etwa 50% Feststoffe hat;
Kombinieren der ersten organischen Phase mit einer ersten wässrigen Phase unter Bildung einer Emulsionsphase;
Kombinieren der Emulsionsphase mit einem Quenchmittel, um eine gequenchte Phase zu bilden, die die Vielzahl therapeutischer Nanopartikel umfasst, wobei das Quenchmittel Wasser und eine im Wesentlichen hydrophobe Säure, die wenigstens etwas Wasserlöslichkeit hat, umfasst; und
Gewinnen der therapeutischen Nanopartikel durch Filtration.

## Revendications

1. Procédé de préparation d'une pluralité de nanoparticules thérapeutiques, comprenant :
la combinaison d'un premier polymère et d'un agent thérapeutique avec un solvant organique, et éventuellement un premier acide sensiblement hydrophobe, pour former une première phase organique ayant d'environ 1 à environ 50 % de solides ;
la combinaison de la première phase organique avec une première phase aqueuse pour former une phase en émulsion ;
la combinaison de la phase en émulsion avec un bain de désactivation pour former une phase désactivée comprenant la pluralité de nanoparticules thérapeutiques, dans laquelle le bain de désactivation comprend de l'eau et un deuxième acide sensiblement hydrophobe ayant au moins une certaine solubilité dans l'eau ; et
la récupération des nanoparticules thérapeutiques par filtration.

2. Procédé selon la revendication 1, dans lequel le premier ou deuxième acide sensiblement hydrophobe est indépendamment choisi dans le groupe constitué par l'acide caproïque, l'acide énanthique, l'acide caprylique, l'acide pélargonique, l'acide caprique, l'acide undécanoïque, l'acide laurique, l'acide tridécanoïque, l'acide myristique, l'acide pentadécanoïque, l'acide palmitique, l'acide cinnamique, l'acide phénylacétique, l'acide dodécylbenzènesulfonique, l'acide dioctylsulfosuccinique, l'acide dioléoylphosphatidique et l'acide chénodésoxycholique, l'acide ursodésoxycholique, l'acide désoxycholique, l'acide cholique, et l'acide lithocholique, et leurs combinaisons.

3. Procédé selon la revendication 1 ou 2, dans lequel le rapport molaire de l'acide sensiblement hydrophobe à l'agent thérapeutique dans la nanoparticule est d'environ 0,1/1 à environ 1,2/1.

4. Procédé selon l'une quelconque des revendications 1 et 2, dans lequel le rapport molaire de l'acide sensiblement hydrophobe à l'agent thérapeutique dans la nanoparticule est d'environ 0,75/1 à environ 1,2/1.

5. Procédé selon l'une quelconque des revendications 1 à 4, dans lequel la première phase organique comprend un acide sensiblement hydrophobe ayant au moins une certaine solubilité dans l'eau.

6. Procédé selon l'une quelconque des revendications 1 à 4, dans lequel la première phase aqueuse comprend un acide sensiblement hydrophobe ayant au moins une certaine solubilité dans l'eau.

7. Procédé selon l'une quelconque des revendications 1 à 6, dans lequel la phase en émulsion comprend un acide sensiblement hydrophobe ayant au moins une certaine solubilité dans l'eau et dans lequel la phase en émulsion comprend un rapport molaire de l'acide sensiblement hydrophobe à l'agent thérapeutique d'environ 0,5/1 à environ 6/1.

8. Procédé selon l'une quelconque des revendications 1 à 7, dans lequel le procédé a un rendement d'encapsulation compris entre environ 75 % et environ 100 %.

9. Procédé selon l'une quelconque des revendications 1 à 8, dans lequel les nanoparticules thérapeutiques comprennent d'environ 5 à environ 20 % en poids de l'agent thérapeutique.

10. Procédé selon l'une quelconque des revendications 1 à 8, dans lequel les nanoparticules thérapeutiques comprennent d'environ 10 à environ 30 % en poids de l'agent thérapeutique.

11. Procédé selon l'une quelconque des revendications 1 à 10, dans lequel le bain de désactivation a une température d'environ 0°C à environ 5°C.

12. Procédé selon l'une quelconque des revendications 1 à 11, dans lequel le rapport bain de désactivation/émulsion est d'environ 2/1 à environ 40/1.

13. Procédé selon l'une quelconque des revendications 1 à 12, comprenant en outre l'addition d'un solubilisant de médicament à la phase désactivée pour former une phase solubilisée d'agent thérapeutique non encapsulé.

14. Procédé selon l'une quelconque des revendications 1 à 13, dans lequel les nanoparticules thérapeutiques libèrent pratiquement immédiatement moins d'environ 5 % de l'agent thérapeutique quand elles sont placées dans une solution de tampon phosphate à 25°C.

15. Pluralité de nanoparticules thérapeutiques préparées par un procédé comprenant :
la combinaison d'un premier polymère et d'un agent thérapeutique avec un solvant organique pour former une première phase organique ayant d'environ 1 à environ 50 % de solides ;
la combinaison de la première phase organique avec une première phase aqueuse pour former une phase en émulsion ;
la combinaison de la phase en émulsion avec un bain de désactivation pour former une phase désactivée comprenant la pluralité de nanoparticules thérapeutiques, dans laquelle le bain de désactivation comprend de l'eau et un acide sensiblement hydrophobe ayant au moins une certaine solubilité dans l'eau ; et
la récupération des nanoparticules thérapeutiques par filtration.
